# EUROPEAN PATENT APPLICATION

(11) **EP 4 549 462 A1**
(43) Date of publication of application: **07.05.2025**
(21) Application number: 24172967.2
(22) Date of filing: 29.04.2024
(51) Int. Cl.: C07K 16/18, G01N 33/00, A61K 39/00

(54) **PROCOLLAGEN I N-TERMINAL PROPEPTIDE-SPECIFIC ANTIBODY, KIT AND USE THEREOF**

(30) Priority: 28.04.2023 CN 202310484959
(71) Applicant: Shenzhen Mindray Bio-Medical Electronics Co., Ltd., Shenzhen, Guangdong 518057 (CN); Hytest Ltd., 20520 Turku (FI)
(72) Inventor: FILATOV, Vladimir L., 20520 Turku (FI); DATSKEVICH, Petr N., 20520 Turku (FI); KOKISHEVA, Antonina S., 20520 Turku (FI); KATRUKHA, Alexey G., 20520 Turku (FI); KOZLOVSKY, Stanislav V., 20520 Turku (FI); POSTNIKOV, Alexander B., 20520 Turku (FI); ROZOV, Fedor N., 20520 Turku (FI); TANG, Tao, Shenzhen, 518057 (CN); Zhan, Chengxiong, Shenzhen, 518057 (CN); WU, Lingjia, Shenzhen, 518057 (CN); ZHU, Shibo, Shenzhen, 518057 (CN); LI, Ke, Shenzhen, 518057 (CN); WU, Yue, Shenzhen, 518057 (CN)
(74) Representative: KIPA AB

(57) **Abstract**

The present invention relates to a PINP-specific antibody, kit and use thereof. Specifically, the present invention provides a PINP-specific antibody, as well as a kit containing the antibody, and use thereof in detecting the presence or level of tPINP in a sample.

## Description

### Technical Field

The present invention belongs to the field of molecular immunology, and specifically relates to a Procollagen I N-Terminal Propeptide-specific antibody, a kit comprising the antibody and use thereof.

### Background Art

Collagens are the most abundant protein in mammals. There are 28 types of collagen. Collagen type I is a trimeric protein found predominantly in bone tissues and in some soft tissues. Procollagen chain comprises amino-terminal and carboxyl-terminal propeptides that are cleaved off by specific proteases during collagen maturation. Both propeptides levels have diagnostic implications. human Procollagen I N-Terminal Propeptide-specific antibody (PINP) increased levels suggest intensified bone formation process such as during osteoporosis treatment or during bone metastasis in cancer.

A method for measurement of Procollagen I N-Terminal Propeptide-specific antibody in human serum has been developed (Melkko et al., 1996, Clinical Chemistry, 42:6, 947-954). Polyclonal antibodies were utilized in the assay only trimeric form was detected by said assay although monomeric form of PINP was indeed present in the blood. Sensitivity of assay was also not sufficient. Therefore, there is still a need to detect both forms of PINP with high sensitivity.

### Contents of the present invention

After extensive experimental research, the inventors of the present invention developed PINP antibodies with excellent performance, and unexpectedly discovered antibody pairs of the PINP antibodies that are particularly suitable for the detection of PINP by double-antibody sandwich method. On this basis, the inventors developed a new PINP quantitative detection kit and detection method. The sensitivity of the antibody (pair), kit or detection method of the present invention to tPINP (total Procollagen I N-Terminal Propeptide) can reach 0.3 ng/ml, and the detection range can reach 0.3 to 2000 ng/ml. In some embodiments, the sensitivity to tPINP can reach 0.03 ng/mg, and the detection range can reach 0.03 to 2000 ng/ml. In addition, the antibody (pair), kit or detection method of the present invention has strong anti-interference ability and can shield the interference of endogenous substances and common PINP analogues in serum. Moreover, the present invention can achieve rapid detection with a smaller sample amount and thus has great clinical application value.

### Antibody

The inventors screened and obtained a class of antibodies that can specifically bind to PINP, have strong binding activity to PINP, and have KD values at the nM level, or even lower, which are provided as new antibody raw materials for the preparation of high-performance PINP detection kits.

Therefore, in one aspect, the present invention provides an antibody or antigen-binding fragment thereof that specifically binds to PINP (Procollagen I N-Terminal Propeptide) or different forms of existence thereof, comprising:
a heavy chain variable region comprising the following CDRs: CDR-H1 having an amino acid sequence selected from SEQ ID NO: 1, 7, 13, 19, 25, 31 and 37 or a variant thereof, CDR-H2 having an amino acid sequence selected from SEQ ID NO: 2, 8, 14, 20, 26, 32 and 38 or a variant thereof, and/or CDR-H3 having an amino acid sequence selected from SEQ ID NO: 3, 9, 15, 21, 27, 33 and 39 or a variant thereof; and/or,
a light chain variable region comprising following three CDRs: CDR-L1 having an amino acid sequence selected from SEQ ID NO: 4, 10, 16, 22, 28, 34 and 40 or a variant thereof, CDR-L2 having an amino acid sequence selected from SEQ ID NO: 5, 11, 17, 23, 29, 35 and 41 or a variant thereof, and/or CDR-L3 having an amino acid sequence selected from SEQ ID NO: 6, 12, 18, 24, 30, 36 and 42 or a variant thereof;
wherein, each variant contains an amino acid mutation, the mutation is substitution, deletion or addition of one or more amino acids (for example, the substitution, deletion or addition of 1, 2, 3, 4 or 5 amino acids) as compared to an amino acid sequence from which said variant is derived; preferably, the substitution is a conservative substitution; preferably, said variant has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which said variant is derived.

In some embodiments, the substitution is a conservative substitution.

In some embodiments, the variant has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which it is derived.

In some embodiments, the antibody or antigen-binding fragment thereof containing the variant is still capable of specifically binding to PINP.

In some embodiments, the three CDRs of the heavy chain variable region, and/or the three CDRs of the light chain variable region is defined by Kabat, Chothia, or IMGT numbering system.

In some embodiments, the antibody or antigen-binding fragment thereof comprises:
(1a) the heavy chain variable region comprises following three CDRs: CDR-H1 having an amino acid sequence of SEQ ID NO: 1 or a variant thereof, CDR-H2 having an amino acid of SEQ ID NO: 2 or a variant thereof, and CDR-H3 having an amino acid sequence of SEQ ID NO: 3 or a variant thereof; and the light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;
(1b) the heavy chain variable region comprises following three CDRs: CDR-H1 having an amino acid sequence of SEQ ID NO: 7 or a variant thereof, CDR-H2 having an amino acid of SEQ ID NO: 8 or a variant thereof, and CDR-H3 having an amino acid sequence of SEQ ID NO: 9 or a variant thereof; and the light chain variable region comprises following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;
(1c) the heavy chain variable region comprises the following three CDRs: CDR-H1 having an amino acid sequence of SEQ ID NO: 13 or a variant thereof, CDR-H2 having an amino acid of SEQ ID NO: 14 or a variant thereof, and CDR-H3 having an amino acid sequence of SEQ ID NO: 15 or a variant thereof; and the light chain variable region comprises following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;
(1d) the heavy chain variable region comprises following three CDRs: CDR-H1 having an amino acid sequence of SEQ ID NO: 19 or a variant thereof, CDR-H2 having an amino acid of SEQ ID NO: 20 or a variant thereof, and CDR-H3 having an amino acid sequence of SEQ ID NO: 21 or a variant thereof; and the light chain variable region comprises following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;
(1e) the heavy chain variable region comprises three CDRs: CDR-H1 having an amino acid sequence of SEQ ID NO: 25 or a variant thereof, CDR-H2 having an amino acid of SEQ ID NO: 26 or a variant thereof, and CDR-H3 having an amino acid sequence of SEQ ID NO: 27 or a variant thereof; and the light chain variable region comprises three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof;
(1f) the heavy chain variable region comprises following three CDRs: CDR-H1 having an amino acid sequence of SEQ ID NO: 31 or a variant thereof, CDR-H2 having an amino acid of SEQ ID NO: 32 or a variant thereof, and CDR-H3 having an amino acid sequence of SEQ ID NO: 33 or a variant thereof; and the light chain variable region comprises following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof;
   or
(1g) the heavy chain variable region comprises following three CDRs: CDR-H1 having an amino acid sequence of SEQ ID NO: 37 or a variant thereof, CDR-H2 having an amino acid of SEQ ID NO: 38 or a variant thereof, and CDR-H3 having an amino acid sequence of SEQ ID NO: 39 or a variant thereof; and the light chain variable region comprises following three CDRs: CDR-L1 having an amino acid sequence of SEQ ID NO: 40 or a variant thereof, CDR-L2 having an amino acid sequence of SEQ ID NO: 41 or a variant thereof, and CDR-L3 having an amino acid sequence of SEQ ID NO: 42 or a variant thereof;
wherein, each variant contains an amino acid mutation, the mutation is substitution, deletion or addition of one or more amino acids (for example, the substitution, deletion or addition of 1, 2, 3, 4 or 5 amino acids) as compared to an amino acid sequence from which said variant is derived.

In some embodiments, the antibody or antigen-binding fragment thereof as described in the above item (1a) : (a1) is produced by a hybridoma cell line deposited with the All-Russian National Collection of Industrial Microorganisms (VKPM) having Accession Number H-214; (a2) comprises same amino acid sequences as those of CDR-H1, CDR-H2 and CDR-H3 of a heavy chain variable region and/or those of CDR-L1, CDR-L2 and CDR-L3 of a light chain variable region of an antibody produced by the hybridoma cell line deposited with VKPM having Accession Number H-214; (a3) comprises same amino acid sequence as that of a heavy chain variable region and/or that of a light chain variable region of an antibody produced by the hybridoma cell line deposited with VKPM having Accession Number H-214; or (a4) comprise same amino acid sequence as that of a heavy chain and/or that of a light chain of an antibody produced by the hybridoma cell line deposited with VKPM having Accession Number H-214.

The deposition date of the hybridoma cell line is February 21, 2024.

In some embodiments, the antibody or antigen-binding fragment thereof as described in the above item (1b): (b1) comprises same amino acid sequence as an amino acid sequence encoded by an inserted portion of plasmid deposited with VKPM having Accession Number B-14744 and/or an amino acid sequence encoded by an inserted portion of plasmid deposited with VKPM having Accession Number B-14745; (b2) of which the heavy chain comprises same amino acid sequence as an amino acid sequence encoded by an inserted of plasmid deposited with VKPM having Accession Number B-14744 and/or the light chain comprises same amino acid sequence as an amino acid sequence encoded by an inserted portion of plasmid deposited with VKPM having Accession Number B-14745; or (b3) comprises same amino acid sequences as those of CDR-H1, CDR-H2 and CDR-H3 set forth in an amino acid sequence encoded by an inserted portion of plasmid deposited with VKPM having Accession Number B-14744 and/or those of CDR-L1, CDR-L2 and CDR-L3 set forth in an amino acid sequence encoded by an inserted portion of plasmid deposited with VKPM having Accession Number B-14745.

In some embodiments, the antibody or antigen-binding fragment thereof as described in the above item (1c): (c1) comprises same amino acid sequence as an amino acid sequence encoded by an inserted portion of plasmid deposited with VKPM having Accession Number B-14746 and/or an amino acid sequence encoded by an inserted portion of plasmid deposited with VKPM having Accession Number B-14747; (c2) of which the heavy chain comprises same amino acid sequence as an amino acid sequence encoded by an inserted of plasmid deposited with VKPM having Accession Number B-14746 and/or the light chain comprises same amino acid sequence as an amino acid sequence encoded by an inserted portion of plasmid deposited with VKPM having Accession Number B-14747; or (c3) comprises same amino acid sequences as those of CDR-H1, CDR-H2 and CDR-H3 set forth in an amino acid sequence encoded by an inserted portion of plasmid deposited with VKPM having Accession Number B-14746 and/or those of CDR-L1, CDR-L2 and CDR-L3 set forth in an amino acid sequence encoded by an inserted portion of plasmid deposited with VKPM having Accession Number B-14747.

In some embodiments, the antibody or antigen-binding fragment thereof as described in the above item (1d): (d1) comprises same amino acid sequence as an amino acid sequence encoded by an inserted portion of plasmid deposited with VKPM having Accession Number B-14748 and/or an amino acid sequence encoded by an inserted portion of plasmid deposited with VKPM having Accession Number B-14749; (d2) of which the heavy chain comprises same amino acid sequence as an amino acid sequence encoded by an inserted of plasmid deposited with VKPM having Accession Number B-14748 and/or the light chain comprises same amino acid sequence as an amino acid sequence encoded by an inserted portion of plasmid deposited with VKPM having Accession Number B-14749; or (d3) comprises same amino acid sequences as those of CDR-H1, CDR-H2 and CDR-H3 set forth in an amino acid sequence encoded by an inserted portion of plasmid deposited with VKPM having Accession Number B-14748 and/or those of CDR-L1, CDR-L2 and CDR-L3 set forth in an amino acid sequence encoded by an inserted portion of plasmid deposited with VKPM having Accession Number B-14749.

In some embodiments, the antibody or antigen-binding fragment thereof as described in the above item (1e): (e1) comprises same amino acid sequence as an amino acid sequence encoded by an inserted portion of plasmid deposited with VKPM having Accession Number B-14750 and/or an amino acid sequence encoded by an inserted portion of plasmid deposited with VKPM having Accession Number B-14751; (e2) of which the heavy chain comprises same amino acid sequence as an amino acid sequence encoded by an inserted of plasmid deposited with VKPM having Accession Number B-14750 and/or the light chain comprises same amino acid sequence as an amino acid sequence encoded by an inserted portion of plasmid deposited with VKPM having Accession Number B-14751; or (e3) comprises same amino acid sequences as those of CDR-H1, CDR-H2 and CDR-H3 set forth in an amino acid sequence encoded by an inserted portion of plasmid deposited with VKPM having Accession Number B-14750 and/or those of CDR-L1, CDR-L2 and CDR-L3 set forth in an amino acid sequence encoded by an inserted portion of plasmid deposited with VKPM having Accession Number B-14751.

In some embodiments, the antibody or antigen-binding fragment thereof as described in the above item (1f): (f1) comprises same amino acid sequence as an amino acid sequence encoded by an inserted portion of plasmid deposited with VKPM having Accession Number B-14752; or (f2) comprises same amino acid sequences as those of CDR-H1, CDR-H2 and CDR-H3 set forth in an amino acid sequence encoded by an inserted portion of plasmid deposited with VKPM having Accession Number B-14752 and/or those of CDR-L1, CDR-L2 and CDR-L3 set forth in an amino acid sequence encoded by an inserted portion of plasmid deposited with VKPM having Accession Number B-14752.

In some embodiments, the antibody or antigen-binding fragment thereof as described in the above item (1g): (g1) comprises same amino acid sequence as an amino acid sequence encoded by an inserted portion of plasmid deposited with VKPM having Accession Number B-14753; or (g2) comprises same amino acid sequences as those of CDR-H1, CDR-H2 and CDR-H3 set forth in an amino acid sequence encoded by an inserted portion of plasmid deposited with VKPM having Accession Number B-14753 and/or those of CDR-L1, CDR-L2 and CDR-L3 set forth in an amino acid sequence encoded by an inserted portion of plasmid deposited with VKPM having Accession Number B-14753.

The deposition date of all of the plasmids described above are March 25, 2024.

In some embodiments, the substitution is a conservative substitution.

In some embodiment, said variant has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which said variant is derived.

In some embodiments, the antibody or antigen-binding fragment thereof containing the variant is still capable of specifically binding to PINP.

In some embodiments, the three CDRs contained in the heavy chain variable region, and/or the three CDRs contained in the light chain variable region, are defined by the Kabat, Chothia, or IMGT numbering system.

In some embodiments, the antibody or antigen-binding fragment thereof further comprises a heavy chain constant region (CH) and a light chain constant region (CL).

In some embodiments, the heavy chain constant region is a mouse heavy chain constant region and the light chain constant region is a mouse light chain constant region.

In some embodiments, the heavy chain constant region is a human heavy chain constant region and the light chain constant region is a human light chain constant region.

In some embodiments, the antibody or antigen-binding fragment thereof is IgG, IgM, IgE, IgD, or IgA.

In some embodiments, the heavy chain constant region is an IgG heavy chain constant region (for example, an IgG1, IgG2, IgG3 or IgG4 heavy chain constant region);

In some embodiments, the light chain constant region is a κ or λ light chain constant region (for example, a human λ light chain constant region).

In some embodiments, the antigen-binding fragment thereof is selected from scFv, Fab, Fab', (Fab')₂, Fv fragment, disulfide bond-linked Fv(dsFv), diabody, bispecific antibody and multi-specific antibody; and/or, the antibody is a murine antibody, chimeric antibody or humanized antibody.

In some embodiments, the antibody or antigen-binding fragment thereof has a detectable label.

As used herein, the detectable label may be any substance detectable by fluorescent, spectroscopic, photochemical, biochemical, immunological, electrical, optical or chemical means. It is particularly preferred that such labels are suitable for immunological detection (e.g., enzyme-linked immunoassay, radioimmunoassay, fluorescent immunoassay, chemiluminescence immunoassay, etc.). Such labels are well known in the art and include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C or ³²P), fluorescent dyes (e.g., fluorescein isothiocyanate (FITC), fluorescein, tetramethylrhodamine isothiocyanate (TRITC), phycoerythrin (PE), Texas red, rhodamine, quantum dots or cyanine dye derivatives (e.g., Cy7, Alexa 750), chemiluminescence (e.g., acridinium esters), and biotin for binding avidin (e.g., streptavidin) modified by the above-mentioned labels. Labels encompassed by the present invention can be detected by methods known in the art. For example, radioactive labels can be detected using photographic film or scintillation calculators, and fluorescent labels can be detected using photodetectors to detect the emitted light. Enzyme labels are generally detected by providing a substrate for enzyme and detecting the product produced by the action the enzyme on the substrate. Thermometric labels are detected by simply visualizing a colored label. Chemiluminescence (e.g., acridinium esters) are generally detected by providing an excitation solution and/or a catalyst to the luminescent substance and detecting the emitted light. Biotin is generally detected by providing an avidin (e.g., streptavidin) modified with the above label and detecting the label on the avidin linked to biotin. In certain embodiments, the detectable label as described above can be linked to the antibody or antigen-binding fragment thereof of the present invention via a linker of different lengths to reduce potential steric hindrance.

In some embodiments, the label is selected from the group consisting of froufluorescein, chemiluminescence (for example, acridinium esters), enzyme (for example, horseradish peroxidase, alkaline phosphatase), radioisotope, biotin, colloidal gold, and magnetic particles.

In some embodiments, the antibody or antigen-binding fragment thereof that specifically binds to PINP or different forms of existence thereof, or the antibody or antigen-binding fragment thereof according to any one of the above items specifically binds to an epitope contained in a sequence of amino acid residues 23 to 161 of COL1A1 (human Collagen I α1).

In some embodiments, the different forms of existence of PINP include monomeric forms of PINP.

### Isolated nucleic acid molecule, expression vector and host cell

The present invention further provides an isolated nucleic acid molecule encoding the antibody or antigen-binding fragment thereof.

The present invention further provides an expression vector, comprising the isolated nucleic acid molecule. In some embodiments, the vector is a plasmid, a virus, a bacteriophage, a bacterium or a viroid.

The present invention further provides a host cell, comprising the abovementioned isolated nucleic acid molecule or expression vector.

In some embodiments, the host cell is an eukaryotic cell, preferably a mammalian cell.

In some embodiments, the host cell is a prokaryotic cell, preferably a colibacillus.

### Kit

For pregnant women with vitamin D deficiency during pregnancy, umbilical cord blood is often used to detect tPINP to evaluate the activity of fetal bone osteoblasts. However, the content of tPINP in umbilical cord blood is only about 0.3 ng/ml, and the detection sensitivity of existing technology cannot meet this demand. For children, especially those less than 1 year old, the bone development is often assessed by detecting tPINP in the blood, while the content of tPINP in the blood of children is often as high as 2000 ng/ml, so the detection range of existing technology may not be sufficient to special groups such as young children. Whether in detection of umbilical cord blood or detection of children's specimens, the amount of blood collected in clinical practice is relatively small, while the sample size required by the current mainstream detection methods is basically greater than 20 µL.

In order to solve the above technical problems, based on the monoclonal antibody of the present invention, an antibody pair that is particularly suitable for PINP double-antibody sandwich detection is screened and obtained.

Therefore, in another aspect, the present invention provides a composition, which comprises the antibody or antigen-binding fragment thereof as described in any one of the preceding items.

In some embodiments, the composition comprises:
a first antibody, which is selected from the antibody or antigen-binding fragment thereof as described in any one of the preceding items, and,
a second antibody, which is selected from the antibody or antigen-binding fragment thereof as described in any one of the preceding items;
wherein, the first antibody and the second antibody target different epitopes of PINP, respectively.

In some embodiments, the first antibody is the antibody or antigen-binding fragment thereof as described in the aforementioned item (1a);
the second antibody is selected from the group of the following antibodies or antigen-binding fragments thereof as described in the preceding items: (1b), (1c), (1d), (1e), (1f) and (1g), preferably (1b), (1d), (1e) or (1f), more preferably (1b), (1d) or (1f).

In some embodiments, the first antibody is selected from the group consisting of the following antibodies or antigen-binding fragments thereof as defined in the preceding items (a1) to (a4); the second antibody is selected from the group consisting of the following antibodies or antigen-binding fragments thereof as defined in the preceding items (b1) to (b3), (d1) to (d3), (e1) to (e3) and (f1) to (f2).

In some embodiments, the first antibody is the antibody or antigen-binding fragment thereof as described in the preceding item (1b);
the second antibody is selected from the group of the following antibodies or antigen-binding fragments thereof as described in the preceding items: (1a), (1c), (1d), (1e), (1f) and (1g), preferably (1a), (1c)), (1d), (1e) or (1g), more preferably (1g).

In some embodiments, the first antibody is selected from the group consisting of the following antibodies or antigen-binding fragments thereof as defined in the preceding items (b1) to (b3); the second antibody is selected from the group consisting of the following antibodies or antigen-binding fragments thereof as defined in the preceding items (a1) to (a4), (c1) to (c3), (d1) to (d3), (e1) to (e3) and (g1) to (g2).

In some embodiments, the first antibody is the antibody or antigen-binding fragment thereof as described in the aforementioned item (1c);
the second antibody is selected from the group of the following antibodies or antigen-binding fragments thereof as described in the preceding items: (1a), (1b), (1d), (1e), (1f) and (1g), preferably (1b), (1e)) or (1f).

In some embodiments, the first antibody is selected from the group consisting of the following antibodies or antigen-binding fragments thereof as defined in the preceding items (c1) to (c3); the second antibody is selected from the group consisting of the following antibodies or antigen-binding fragments thereof as defined in the preceding items (b1) to (b3), (e1) to (e3) and (f1) to (f2).

In some embodiments, the first antibody is the antibody or antigen-binding fragment thereof as described in the aforementioned item (1d);
the second antibody is selected from the group of the following antibodies or antigen-binding fragments thereof as described in the preceding items: (1a), (1b), (1c), (1e), (1f) and (1g), preferably (1a), (1b) or (1f).

In some embodiments, the first antibody is selected from the group consisting of the following antibodies or antigen-binding fragments thereof as defined in the preceding items (d1) to (d3); the second antibody is selected from the group consisting of the following antibodies or antigen-binding fragments thereof as defined in the preceding items (a1) to (a4), (b1) to (b3) and (f1) to (f2).

In some embodiments, the first antibody is the antibody or antigen-binding fragment thereof as described in the aforementioned item (1e);
the second antibody is selected from the group of the following antibodies or antigen-binding fragments thereof as described in the preceding items: (1a), (1b), (1c), (1d), (1f) and (1g), preferably (1b), (1c)) or (1f), more preferably (1b).

In some embodiments, the first antibody is selected from the group consisting of the following antibodies or antigen-binding fragments thereof as defined in the preceding items (e1) to (e3); the second antibody is selected from the group consisting of the following antibodies or antigen-binding fragments thereof as defined in the preceding items (b1) to (b3), (c1) to (c3) and (f1) to (f2).

In some embodiments, the first antibody is the antibody or antigen-binding fragment thereof as described in the aforementioned item (1f);
the second antibody is selected from the antibodies or antigen-binding fragments thereof as described in the preceding items as follows: (1a), (1b), (1c), (1d), (1e) and (1g).

In some embodiments, the first antibody is the antibody or antigen-binding fragment thereof as described in the aforementioned item (1g);
the second antibody is selected from the group of the following antibodies or antigen-binding fragments thereof as described in the preceding items: (1a), (1b), (1c), (1d), (1e) and (1f).

In some embodiments, the composition comprises:
the first antibody as described in item (1a) above (e.g., the antibody or antigen-binding fragment thereof as described in the preceding items (a1) to (a4)), and the second antibody as described in item (1b) above (e.g., the antibody or antigen-binding fragment thereof as described in the preceding items (b1) to (b3));
the first antibody as described in item (1a) above (e.g., the antibody or antigen-binding fragment thereof as described in the preceding items (a1) to (a4)), and the second antibody as described in item (1d) above (e.g., the antibody or antigen-binding fragment thereof as described in the preceding items (d1) to (d3));
the first antibody as described in item (1a) above (e.g., the antibody or antigen-binding fragment thereof as described in the preceding items (a1) to (a4)), and the second antibody as described in item (1e) above (e.g., the antibody or antigen-binding fragment thereof as described in the preceding items (e1) to (e3));
the first antibody as described in item (1a) above (e.g., the antibody or antigen-binding fragment thereof as described in the preceding items (a1) to (a4)), and the second antibody as described in item (1f) above (e.g., the antibody or antigen-binding fragment thereof as described in the preceding items (f1) to (f3));
the first antibody as described in item (1b) above (e.g., the antibody or antigen-binding fragment thereof as described in the preceding items (b1) to (b3)), and the second antibody as described in item (1a) above (e.g., the antibody or antigen-binding fragment thereof as described in the preceding items (a1) to (a4));
the first antibody as described in item (1b) above (e.g., the antibody or antigen-binding fragment thereof as described in the preceding items (b1) to (b3)), and the second antibody as described in item (1c) above (e.g., the antibody or antigen-binding fragment thereof as described in the preceding items (c1) to (c3));
the first antibody as described in item (1b) above (e.g., the antibody or antigen-binding fragment thereof as described in the preceding items (b1) to (b3)), and the second antibody as described in item (1d) above (e.g., the antibody or antigen-binding fragment thereof as described in the preceding items (d1) to (d3));
the first antibody as described in item (1b) above (e.g., the antibody or antigen-binding fragment thereof as described in the preceding items (b1) to (b3)), and the second antibody as described in item (1e) above (e.g., the antibody or antigen-binding fragment thereof as described in the preceding items (e1) to (e3));
the first antibody as described in item (1b) above (e.g., the antibody or antigen-binding fragment thereof as described in the preceding items (b1) to (b3)), and the second antibody as described in item (1g) above (e.g., the antibody or antigen-binding fragment thereof as described in the preceding items (g1) to (g2));
the first antibody as described in item (1c) above (e.g., the antibody or antigen-binding fragment thereof as described in the preceding items (c1) to (c3)), and the second antibody as described in item (1b) above (e.g., the antibody or antigen-binding fragment thereof as described in the preceding items (b1) to (b3));
the first antibody as described in item (1c) above (e.g., the antibody or antigen-binding fragment thereof as described in the preceding items (c1) to (c3)), and the second antibody as described in item (1e) above (e.g., the antibody or antigen-binding fragment thereof as described in the preceding items (e1) to (e3));
the first antibody as described in item (1c) above (e.g., the antibody or antigen-binding fragment thereof as described in the preceding items (c1) to (c3)), and the second antibody as described in item (1f) above (e.g., the antibody or antigen-binding fragment thereof as described in the preceding items (f1) to (f3));
the first antibody as described in item (1d) above (e.g., the antibody or antigen-binding fragment thereof as described in the preceding items (d1) to (d3)), and the second antibody as described in item (1a) (e.g., the antibody or antigen-binding fragment thereof as described in the preceding items (a1) to (a4));
the first antibody as described in item (1d) above (e.g., the antibody or antigen-binding fragment thereof as described in the preceding items (d1) to (d3)), and the second antibody as described in item (1b) (e.g., the antibody or antigen-binding fragment thereof as described in the preceding items (b1) to (b3));
the first antibody as described in item (1d) above (e.g., the antibody or antigen-binding fragment thereof as described in the preceding items (d1) to (d3)), and the second antibody as described in item (1f) (e.g., the antibody or antigen-binding fragment thereof as described in the preceding items (f1) to (f3));the first antibody as described in item (1e) above (e.g., the antibody or antigen-binding fragment thereof as described in the preceding items (e1) to (e3)), and the second antibody as described in item (1b) above (e.g., the antibody or antigen-binding fragment thereof as described in the preceding items (b1) to (b3));
the first antibody as described in item (1e) above (e.g., the antibody or antigen-binding fragment thereof as described in the preceding items (e1) to (e3)), and the second antibody as described in item (1c) above (e.g., the antibody or antigen-binding fragment thereof as described in the preceding items (c1) to (c3)); or
the first antibody as described in item (1e) above (e.g., the antibody or antigen-binding fragment thereof as described in the preceding items (e1) to (e3)), and the second antibody as described in item (1b) above (e.g., the antibody or antigen-binding fragment thereof as described in the preceding items (f1) to (f2)).

On this basis, the present invention further provides a kit and a detection method with high sensitivity and a wide detection range to meet specific clinical needs.

In another aspect, the present invention provides a PINP detection kit, which has a tPINP detection sensitivity of ≤0.3ng/ml, such as ≤0.2ng/ml, ≤0.15ng/ml, ≤0.1ng/ml, ≤0.05ng/ml, or ≤0.03ng/ml.

In another aspect, the present invention provides a PINP detection kit, which has a tPINP detection range of 0.3 to 2000 ng/ml, such as 0.05 to 2000 ng/ml, 0.1 to 2000 ng/ml, or 0.15 to 2000 ng/ml.

In another aspect, the present invention provides a PINP detection kit, which comprises the antibody or antigen-binding fragment thereof as described in any one of the preceding items.

In some embodiments, the kit comprises:
a first antibody, which is selected from the antibody or antigen-binding fragment thereof as described in any one of preceding items, and,
a second antibody, which is selected from the antibody or antigen-binding fragment thereof as described in any one of preceding items;
preferably, the first antibody and the second antibody target different epitopes of PINP, respectively.

In some embodiments, the first antibody is the antibody or antigen-binding fragment thereof as described in the aforementioned item (1a);
the second antibody is selected from the group of the following antibodies or antigen-binding fragments thereof as described in the preceding items: (1b), (1c), (1d), (1e), (1f) and (1g), preferably is (1b), (1d)), (1e) or (1f), more preferably is (1b), (1d) or (1f).

In some embodiments, the first antibody is selected from the group consisting of the following antibodies or antigen-binding fragments thereof as defined in preceding items (a1) to (a4); the second antibody is selected from the group consisting of the following antibodies or antigen-binding fragments thereof as defined in preceding items (b1) to (b3), (d1) to (d3), (e1) to (e3) and (f1) to (f2).

In some embodiments, the first antibody is the antibody or antigen-binding fragment thereof as described in the aforementioned item (1b);
the second antibody is selected from the group of the following antibodies or antigen-binding fragments thereof as described in the preceding items: (1a), (1c), (1d), (1e), (1f) and (1g), preferably is (1a), (1c)), (1d), (1e) or (1g), more preferably is (1g).

In some embodiments, the first antibody is selected from the group consisting of the following antibodies or antigen-binding fragments thereof as defined in preceding items (b1) to (b3); the second antibody is selected from the group consisting of the following antibodies or antigen-binding fragments thereof as defined in preceding items (a1) to (a4), (c1) to (c3), (d1) to (d3), (e1) to (e3) and (g1) to (g2).

In some embodiments, the first antibody is the antibody or antigen-binding fragment thereof as described in the aforementioned item (1c);
the second antibody is selected from the group of the following antibodies or antigen-binding fragments thereof as described in the preceding items: (1a), (1b), (1d), (1e), (1f) and (1g), preferably is (1b), (1e) or (1f).

In some embodiments, the first antibody is selected from the group consisting of the following antibodies or antigen-binding fragments thereof as defined in preceding items (c1) to (c3); the second antibody is selected from the group consisting of the following antibodies or antigen-binding fragments thereof as defined in preceding items (b1) to (b3), (e1) to (e3) and (f1) to (f2).

In some embodiments, the first antibody is the antibody or antigen-binding fragment thereof as described in the aforementioned item (1d);
the second antibody is selected from the group of the following antibodies or antigen-binding fragments thereof as described in the preceding items: (1a), (1b), (1c), (1e), (1f) and (1g), preferably is (1a), (1b)) or (1f).

In some embodiments, the first antibody is selected from the group consisting of the following antibodies or antigen-binding fragments thereof as defined in preceding items (d1) to (d3); the second antibody is selected from the group consisting of the following antibodies or antigen-binding fragments thereof as defined in preceding items (a1) to (a4), (b1) to (b3) and (f1) to (f2).

In some embodiments, the first antibody is the antibody or antigen-binding fragment thereof as described in the aforementioned item (1e);
the second antibody is selected from the group of the following antibodies or antigen-binding fragments thereof as described in the preceding items: (1a), (1b), (1c), (1d), (1f) and (1g), preferably is (1b), (1c)) or (1f), more preferably is (1b).

In some embodiments, the first antibody is selected from the group consisting of the following antibodies or antigen-binding fragments thereof as defined in preceding items (e1) to (e3); the second antibody is selected from the group consisting of the following antibodies or antigen-binding fragments thereof as defined in preceding items (b1) to (b3), (c1) to (c3) and (f1) to (f2).

In some embodiments, the first antibody is the antibody or antigen-binding fragment thereof as described in the aforementioned item (1f);
the second antibody is selected from the group of the following antibodies or antigen-binding fragments thereof as described in the preceding items: (1a), (1b), (1c), (1d), (1e) and (1g).

In some embodiments, the first antibody is the antibody or antigen-binding fragment thereof as described in the aforementioned item (1g);
the second antibody is selected from the group of the following antibodies or antigen-binding fragments thereof as described in the preceding items: (1a), (1b), (1c), (1d), (1e) and (1f).

In some embodiments, the kit contains:
the first antibody as described in item (1a) above (e.g., the antibody or antigen-binding fragment thereof as described in the preceding items (a1) to (a4)), and the second antibody as described in item (1b) above (e.g., the antibody or antigen-binding fragment thereof as described in the preceding items (b1) to (b3));
the first antibody as described in item (1a) above (e.g., the antibody or antigen-binding fragment thereof as described in the preceding items (a1) to (a4)), and the second antibody as described in item (1d) above (e.g., the antibody or antigen-binding fragment thereof as described in the preceding items (d1) to (d3));
the first antibody as described in item (1a) above (e.g., the antibody or antigen-binding fragment thereof as described in the preceding items (a1) to (a4)), and the second antibody as described in item (1e) above (e.g., the antibody or antigen-binding fragment thereof as described in the preceding items (e1) to (e3));
the first antibody as described in item (1a) above (e.g., the antibody or antigen-binding fragment thereof as described in the preceding items (a1) to (a4)), and the second antibody as described in item (1f) above (e.g., the antibody or antigen-binding fragment thereof as described in the preceding items (f1) to (f3));
the first antibody as described in item (1b) above (e.g., the antibody or antigen-binding fragment thereof as described in the preceding items (b1) to (b3)), and the second antibody as described in item (1a) above (e.g., the antibody or antigen-binding fragment thereof as described in the preceding items (a1) to (a4));
the first antibody as described in item (1b) above (e.g., the antibody or antigen-binding fragment thereof as described in the preceding items (b1) to (b3)), and the second antibody as described in item (1c) above (e.g., the antibody or antigen-binding fragment thereof as described in the preceding items (c1) to (c3));
the first antibody as described in item (1b) above (e.g., the antibody or antigen-binding fragment thereof as described in the preceding items (b1) to (b3)), and the second antibody as described in item (1d) above (e.g., the antibody or antigen-binding fragment thereof as described in the preceding items (d1) to (d3));
the first antibody as described in item (1b) above (e.g., the antibody or antigen-binding fragment thereof as described in the preceding items (b1) to (b3)), and the second antibody as described in item (1e) above (e.g., the antibody or antigen-binding fragment thereof as described in the preceding items (e1) to (e3));
the first antibody as described in item (1b) above (e.g., the antibody or antigen-binding fragment thereof as described in the preceding items (b1) to (b3)), and the second antibody as described in item (1g) above (e.g., the antibody or antigen-binding fragment thereof as described in the preceding items (g1) to (g2));
the first antibody as described in item (1c) above (e.g., the antibody or antigen-binding fragment thereof as described in the preceding items (c1) to (c3)), and the second antibody as described in item (1b) above (e.g., the antibody or antigen-binding fragment thereof as described in the preceding items (b1) to (b3));
the first antibody as described in item (1c) above (e.g., the antibody or antigen-binding fragment thereof as described in the preceding items (c1) to (c3)), and the second antibody as described in item (1e) above (e.g., the antibody or antigen-binding fragment thereof as described in the preceding items (e1) to (e3));
the first antibody as described in item (1c) above (e.g., the antibody or antigen-binding fragment thereof as described in the preceding items (c1) to (c3)), and the second antibody as described in item (1f) above (e.g., the antibody or antigen-binding fragment thereof as described in the preceding items (f1) to (f3));
the first antibody as described in item (1d) above (e.g., the antibody or antigen-binding fragment thereof as described in the preceding items (d1) to (d3)), and the second antibody as described in item (1a) (e.g., the antibody or antigen-binding fragment thereof as described in the preceding items (a1) to (a4));
the first antibody as described in item (1d) above (e.g., the antibody or antigen-binding fragment thereof as described in the preceding items (d1) to (d3)), and the second antibody as described in item (1b) (e.g., the antibody or antigen-binding fragment thereof as described in the preceding items (b1) to (b3));
the first antibody as described in item (1d) above (e.g., the antibody or antigen-binding fragment thereof as described in the preceding items (d1) to (d3)), and the second antibody as described in item (1f) (e.g., the antibody or antigen-binding fragment thereof as described in the preceding items (f1) to (f3));the first antibody as described in item (1e) above (e.g., the antibody or antigen-binding fragment thereof as described in the preceding items (e1) to (e3)), and the second antibody as described in item (1b) above (e.g., the antibody or antigen-binding fragment thereof as described in the preceding items (b1) to (b3));
the first antibody as described in item (1e) above (e.g., the antibody or antigen-binding fragment thereof as described in the preceding items (e1) to (e3)), and the second antibody as described in item (1c) above (e.g., the antibody or antigen-binding fragment thereof as described in the preceding items (c1) to (c3)); or
the first antibody as described in item (1e) above (e.g., the antibody or antigen-binding fragment thereof as described in the preceding items (e1) to (e3)), and the second antibody as described in item (1b) above (e.g., the antibody or antigen-binding fragment thereof as described in the preceding items (f1) to (f2)).

In some embodiments, the first antibody is a capture antibody and the second antibody is a detection antibody.

In some embodiments, the kit further comprises a solid phase carrier. In certain embodiments, the solid phase carrier includes concave well plates, test tubes, beads (e.g., latex particles) or thin films (e.g. nitrocellulose membranes), which are made of or coated with polymer material (e.g., polyvinyl chloride, polystyrene, polyacrylamide, or cellulose), or magnetic beads pre-coated with functional groups (e.g. amino, carboxyl, biotin or avidin). In certain embodiments, the solid phase carrier is selected from magnetic beads or microtiter plates (e.g., microplates or enzyme plates).

In some embodiments, the capture antibody is coated on the surface of the solid phase carrier.

In some embodiments, the detection antibody has a detectable label.

In some embodiments, the label is selected from the group consisting of fluorescein, chemiluminescence (e.g., acridinium esters), enzymes (e.g., horseradish peroxidase, alkaline phosphatase), radioisotopes, biotin, and colloidal gold.

In some embodiments, the kit may further comprise a reagent that allows the corresponding detectable label to be detected. For example, when the detectable label is an enzyme, the kit may further comprise a chromogenic substrate for the corresponding enzyme, such as o-phenylenediamine (OPD), tetramethylbenzidine (TMB), ABTS or luminol compounds for horseradish peroxidase, or p-nitrophenyl phosphate (p-NPP) or AMPPD for alkaline phosphatase. For example, when the detectable label is a chemiluminescent reagent (e.g., an acridinium ester compound), the kit may further comprise a pre-excitation solution and/or an excitation solution for chemiluminescence.

In some embodiments, the kit further comprises a coating reagent for coating the first antibody (capture antibody) on the solid phase carrier, such as a coating buffer (e.g., carbonate buffer solution, phosphate buffer solution, Tris-HCL buffer solution or borate buffer solution). Methods for coating proteins or polypeptides on solid phase carriers are well known in the art, such as physical adsorption, covalent coupling achieved by aminated or carboxylated surfaces, or mediation binding achieved by avidin-biotin system, polylysine pre-coated surface, Protein A or Protein G pre-coated surface.

In some embodiments, the kit comprises a capture antibody diluent and a detection antibody diluent.

In some embodiments, the capture antibody diluent contains: 10 to 500 mM buffer system, 10 to 500 mM NaCl, 0.01% to 0.1% Proclin300, 0.05% to 0.5% Tween-20, and 0.1% to 5% BSA, pH is 6.0 to 9.0.

In some embodiments, the buffer system is selected from Tris, Hepes, and PBS buffer systems.

In some embodiments, the capture antibody diluent has a pH of 6.0 to 6.5, 6.0 to 7.0, 6.0 to 7.5, 6.0 to 8.0, 6.0 to 8.5, 6.0 to 9.0, 6.5 to 7.0, 6.5 to 7.5, 6.5 to 8.0, 6.5 to 8.5, 6.5 to 9.0, 7.0 to 7.5, 7.0 to 8.0, 7.0 to 8.5, 7.0 to 9.0, 7.5 to 8.0, 7.5 to 8.5, 7.5 to 9.0, 8.0 to 8.5, 8.0 to 9.0 or 8.5 to 9.0.

In some embodiments, the capture antibody diluent comprises: 50 to 200 mM buffer system, 50 to 200 mM NaCl, 0.02% to 0.06% Proclin300, 0.05% to 0.2% Tween-20, and 0.5% to 2% BSA, pH is 6.0 to 9.0.

In some embodiments, the capture antibody diluent consists of: 50 mM Tris, 0.9% NaCl, 0.048% Proclin300, 0.1% Tween-20, 1% BSA, pH 8;
100 mM Hepes, 0.9% NaCl, 0.048% Proclin300, 0.1% Tween-20, 1% BSA, pH 7.4; or
100 mM PBS, 0.9% NaCl, 0.048% Proclin300, 0.1% Tween-20, 1% BSA, pH 7.4.

In some embodiments, the detection antibody diluent comprises: 10 to 500 mM buffer system, 10 to 500 mM NaCl, 0.01% to 0.1% Proclin300, 0.05% to 0.5% Tween-20, and 0.1% to 5% BSA, pH is 6.0 to 9.0.

In some embodiments, the buffer system is selected from Tris, Hepes, and MES buffer systems.

In some embodiments, the detection antibody diluent has a pH of 6.0 to 6.5, 6.0 to 7.0, 6.0 to 7.5, 6.0 to 8.0, 6.0 to 8.5, 6.0 to 9.0, 6.5 to 7.0, 6.5 to 7.5, 6.5 to 8.0, 6.5 to 8.5, 6.5 to 9.0, 7.0 to 7.5, 7.0 to 8.0, 7.0 to 8.5, 7.0 to 9.0, 7.5 to 8.0, 7.5 to 8.5, 7.5 to 9.0, 8.0 to 8.5, 8.0 to 9.0 or 8.5 to 9.0.

In some embodiments, the detection antibody diluent further comprises: 1 to 5 mM MgCl₂ and 0.05 to 0.5 mM ZnCl₂.

In some embodiments, the detection antibody diluent comprises: 50 to 200 mM buffer system, 50 to 200 mM NaCl, 0.02% to 0.06% Proclin300, 0.05% to 0.2% Tween-20, and 0.5% to 2% BSA, and optionally 1 to 3 mM MgCl₂ and 0.05 to 0.2 mM ZnCl₂, pH 6.0 to 9.0.

In some embodiments, the detection antibody diluent consists of: 50 mM Tris, 0.9% NaCl, 0.048% Proclin300, 0.1% Tween-20, 1% BSA, pH 8;
100 mM Hepes, 0.9% NaCl, 0.048% Proclin300, 0.1% Tween-20, 1% BSA, pH 7.4;
50 mM MES, 0.9% NaCl, 0.048% Proclin300, 0.1% Tween-20, 1% BSA, pH 6.0; or
50 mM MES, 0.9% NaCl, 0.048% Proclin300, 0.1% Tween-20, 1% BSA, 2 mM MgCl₂, 0.1mM ZnCl₂, pH 6.0.

In some embodiments, the PINP detection kit comprises: a capture antibody immobilized on magnetic particles, a detection antibody labeled with alkaline phosphatase, a capture antibody diluent and a detection antibody diluent, wherein,
the capture antibody is the antibody or antigen-binding fragment thereof as defined in item (1a) (e.g., the antibody or antigen-binding fragment thereof as described in the preceding items (a1) to (a4)), and the detection antibody is the antibody or antigen-binding fragment thereof as defined in item (1d) (e.g., the antibody or antigen-binding fragment thereof as described in the preceding items (d1) to (d3)); or
the capture antibody is the antibody or antigen-binding fragment thereof as defined in item (1c) (e.g., the antibody or antigen-binding fragment thereof as described in the preceding items (c1) to (c3)), and the detection antibody is the antibody or antigen-binding fragment thereof as defined in item (1f) (e.g., the antibody or antigen-binding fragment thereof as described in the preceding items (f1) to (f2)); or
the capture antibody is the antibody or antigen-binding fragment thereof as defined in item (1e) (e.g., the antibody or antigen-binding fragment thereof as described in the preceding items (e1) to (e3)), and the detection antibody is the antibody or antigen-binding fragment thereof as defined in item (1b) (e.g., the antibody or antigen-binding fragment thereof as described in the preceding items (b1) to (b3)); or
the capture antibody is the antibody or antigen-binding fragment thereof as defined in item (1a) (e.g., the antibody or antigen-binding fragment thereof as described in the preceding items (a1) to (a4)), and the detection antibody is the antibody or antigen-binding fragment thereof as defined in item (1f) (e.g., the antibody or antigen-binding fragment thereof as described in the preceding items (f1) to (f2)); or
the capture antibody is the antibody or antigen-binding fragment thereof as defined in item (1a) (e.g., the antibody or antigen-binding fragment thereof as described in the preceding items (a1) to (a4)), and the detection antibody is the antibody or antigen-binding fragment thereof as defined in item (1b) (e.g., the antibody or antigen-binding fragment thereof as described in the preceding items (b1) to (b3)); or
the capture antibody is the antibody or antigen-binding fragment thereof as defined in item (1c) (e.g., the antibody or antigen-binding fragment thereof as described in the preceding items (c1) to (c3)), and the detection antibody is the antibody or antigen-binding fragment thereof as defined in item (1b) (e.g., the antibody or antigen-binding fragment thereof as described in the preceding items (b1) to (b3)).

In some embodiments, the capture antibody is selected from the group consisting of the following antibodies or antigen-binding fragments thereof as defined in preceding items (a1) to (a4); the detection antibody is selected from the group consisting of the following antibodies or antigen-binding fragments thereof as defined in preceding items (b1) to (b3).

In some embodiments, the capture antibody is selected from the group consisting of the following antibodies or antigen-binding fragments thereof as defined in preceding items (a1) to (a4); the detection antibody is selected from the group consisting of the following antibodies or antigen-binding fragments thereof as defined in preceding items (d1) to (d3).

In some embodiments, the capture antibody is selected from the group consisting of the following antibodies or antigen-binding fragments thereof as defined in preceding items (a1) to (a4); the detection antibody is selected from the group consisting of the following antibodies or antigen-binding fragments thereof as defined in preceding items (f1) to (f2).

In some embodiments, the capture antibody is selected from the group consisting of the following antibodies or antigen-binding fragments thereof as defined in preceding items (c1) to (c3); the detection antibody is selected from the group consisting of the following antibodies or antigen-binding fragments thereof as defined in preceding items (b1) to (b3).

In some embodiments, the capture antibody is selected from the group consisting of the following antibodies or antigen-binding fragments thereof as defined in preceding items (c1) to (c3); the detection antibody is selected from the group consisting of the following antibodies or antigen-binding fragments thereof as defined in preceding items and (f1) to (f2).

In some embodiments, the capture antibody is selected from the group consisting of the following antibodies or antigen-binding fragments thereof as defined in preceding items (e1) to (e3); the detection antibody is selected from the group consisting of the following antibodies or antigen-binding fragments thereof as defined in preceding items (b1) to (b3).In some embodiments, the PINP detection kit comprises: a capture antibody immobilized on magnetic particles, a detection antibody labeled with alkaline phosphatase, a capture antibody diluent and a detection antibody diluent, wherein,
the capture antibody is the antibody or antigen-binding fragment thereof as described in item (1a) above, the detection antibody is the antibody or antigen-binding fragment thereof as described in item (1d) above, the capture antibody diluent is composed of: 50 mM Tris, 0.9% NaCl, 0.048% Proclin300, 0.1% Tween-20, 1% BSA, pH 8, and the detection antibody diluent is composed of: 50 mM Tris, 0.9% NaCl, 0.048% Proclin300, 0.1% Tween-20, 1% BSA, pH 8;
the capture antibody is the antibody or antigen-binding fragment thereof as described in item (1c) above, the detection antibody is the antibody or antigen-binding fragment thereof as described in item (1f) above, and the capture antibody diluent is composed of: 100 mM Hepes, 0.9% NaCl, 0.048% Proclin300, 0.1% Tween-20, 1% BSA, pH 7.4, and the detection antibody diluent is composed of: 100 mM Hepes, 0.9% NaCl, 0.048% Proclin300, 0.1% Tween-20, 1% BSA, pH 7.4; or
the capture antibody is the antibody or antigen-binding fragment thereof as described in item (1e) above, the detection antibody is the antibody or antigen-binding fragment thereof as described in item (1b) above, and the capture antibody diluent is composed of: 100 mM PBS, 0.9% NaCl, 0.048% Proclin300, 0.1% Tween-20, 1% BSA, pH 7.4, and the detection antibody diluent is composed of: 50 mM MES, 0.9% NaCl, 0.048% Proclin300, 0.1% Tween-20, 1% BSA, pH 6.0.

In some embodiments, the kit further comprises one or more of the following: a PINP calibrator, a chemiluminescent substrate, a washing solution, a stop solution, and an user's manual.

In some embodiments, the PINP calibrator comprise the amino acid sequence as set forth in SEQ ID NO: 43.

In some embodiments, the kit is used to detect the presence or level of tPINP, particularly PINP, in a sample.

In some embodiments, the sample is from human, such as human body fluids (e.g., blood, serum, or plasma).

### Detection method and use

In another aspect, the present invention provides a method for detecting the presence or level (concentration) of tPINP, especially PINP, in a sample, comprising detecting by using the antibody or antigen-binding fragment thereof or the PINP detection kit as described in any one of the preceding items.

In some embodiments, the method comprises a step of contacting the sample with two or more antibodies or antigen-binding fragments thereof selected from the antibody or antigen-binding fragment thereof as described in any of preceding items, and qualitatively or quantitatively detecting binding of the two or more antibodies or antigen-binding fragments thereof to PINP and different forms of existence thereof (e.g., PINP monomeric form), the binding indicating the presence or concentration of PINP and different forms of existence thereof in the sample, wherein,
the two or more antibodies or antigen-binding fragments thereof target two or more epitopes of PINP.

In some embodiments, at least one of the two or more antibodies or antigen-binding fragments thereof is a capture antibody and the rest are a detection antibody or detection antibodies.

In some embodiments, the capture antibody and detection antibody are selected from Group 1 to Group 7:
Group 1: the capture antibody is the antibody or antigen-binding fragment thereof as described in item (1a) above;
   the detection antibody is selected from the group of the following antibodies or antigen-binding fragments thereof as described in the preceding items: (1b), (1c), (1d), (1e), (1f) and (1g), preferably (1b), (1d), (1e) or (1f), more preferably (1b), (1d) or (1f);
Group 2: the capture antibody is the antibody or antigen-binding fragment thereof as described in item (1b) above;
   the detection antibody is selected from the group of the following antibodies or antigen-binding fragments thereof as described in the preceding items: (1a), (1c), (1d), (1e), (1f) and (1g), preferably (1a), (1c), (1d), (1e) or (1g), more preferably (1g);
Group 3: the capture antibody is the antibody or antigen-binding fragment thereof as described in item (1c) above;
   the detection antibody is selected from the group of the following antibodies or antigen-binding fragments thereof as described in the preceding items: (1a), (1b), (1d), (1e), (1f) and (1g), preferably (1b), (1e) or (1f);
Group 4: the capture antibody is the antibody or antigen-binding fragment thereof as described in item (1d) above;
   the detection antibody is selected from the group of the following antibodies or antigen-binding fragments thereof as described in the preceding items: (1a), (1b), (1c), (1e), (1f) and (1g), preferably (1a), (1b) or (1f);
Group 5: the capture antibody is the antibody or antigen-binding fragment thereof as described in item (1e) above;
   the detection antibody is selected from the group of the following antibodies or antigen-binding fragments thereof as described in the preceding items: (1a), (1b), (1c), (1d), (1f) and (1g), preferably (1b), (1c) or (1f), more preferably (1b);
Group 6: the capture antibody is the antibody or antigen-binding fragment thereof as described in item (1f) above;
   the detection antibody is selected from the group of the following antibodies or antigen-binding fragments thereof as described in the preceding items: (1a), (1b), (1c), (1d), (1e) and (1g); and
Group 7: the capture antibody is the antibody or antigen-binding fragment thereof as described in item (1g) above;
   the detection antibody is selected from the group of the following antibodies or antigen-binding fragments thereof as described in the preceding items: (1a), (1b), (1c), (1d), (1e) and (1f).

In some embodiments, the capture antibody and detection antibody are selected from:
the capture antibody is the antibody or antigen-binding fragment thereof as defined in item (1a) (e.g., the antibody or antigen-binding fragment thereof as described in the preceding items (a1) to (a4)), and the detection antibody is the antibody or antigen-binding fragment thereof as defined in item (1b) (e.g., the antibody or antigen-binding fragment thereof as described in the preceding items (b1) to (b3));
the capture antibody is the antibody or antigen-binding fragment thereof as defined in item (1a) (e.g., the antibody or antigen-binding fragment thereof as described in the preceding items (a1) to (a4)), and the detection antibody is the antibody or antigen-binding fragment thereof as defined in item (1d) (e.g., the antibody or antigen-binding fragment thereof as described in the preceding items (d1) to (d3));
the capture antibody is the antibody or antigen-binding fragment thereof as defined in item (1a) (e.g., the antibody or antigen-binding fragment thereof as described in the preceding items (a1) to (a4)), and the detection antibody is the antibody or antigen-binding fragment thereof as defined in item (1e) (e.g., the antibody or antigen-binding fragment thereof as described in the preceding items (e1) to (e3));
the capture antibody is the antibody or antigen-binding fragment thereof as defined in item (1a) (e.g., the antibody or antigen-binding fragment thereof as described in the preceding items (a1) to (a4)), and the detection antibody is the antibody or antigen-binding fragment thereof as defined in item (1f) (e.g., the antibody or antigen-binding fragment thereof as described in the preceding items (f1) to (f2));
the capture antibody as described in item (1b) above (e.g., the antibody or antigen-binding fragment thereof as described in the preceding items (b1) to (b3)), and the detection antibody as described in item (1a) above (e.g., the antibody or antigen-binding fragment thereof as described in the preceding items (a1) to (a4));
the capture antibody as described in item (1b) above (e.g., the antibody or antigen-binding fragment thereof as described in the preceding items (b1) to (b3)), and the detection antibody as described in item (1c) above (e.g., the antibody or antigen-binding fragment thereof as described in the preceding items (c1) to (c3));
the capture antibody as described in item (1b) above (e.g., the antibody or antigen-binding fragment thereof as described in the preceding items (b1) to (b3)), and the detection antibody as described in item (1d) above (e.g., the antibody or antigen-binding fragment thereof as described in the preceding items (d1) to (d3));
the capture antibody as described in item (1b) above (e.g., the antibody or antigen-binding fragment thereof as described in the preceding items (b1) to (b3)), and the detection antibody as described in item (1e) above (e.g., the antibody or antigen-binding fragment thereof as described in the preceding items (e1) to (e3));
the antibody or antigen-binding fragment thereof as described in the preceding items (b1) to (b3)), and the detection antibody is the antibody or antigen-binding fragment thereof as defined in item (1g) (e.g., the antibody or antigen-binding fragment thereof as described in the preceding items (g1) to (g2));
the capture antibody is the antibody or antigen-binding fragment thereof as defined in item (1c) (e.g., the antibody or antigen-binding fragment thereof as described in the preceding items (c1) to (c3)), and the detection antibody is the antibody or antigen-binding fragment thereof as defined in item (1b) (e.g., the antibody or antigen-binding fragment thereof as described in the preceding items (b1) to (b3));
the capture antibody is the antibody or antigen-binding fragment thereof as defined in item (1c) (e.g., the antibody or antigen-binding fragment thereof as described in the preceding items (c1) to (c3)), and the detection antibody is the antibody or antigen-binding fragment thereof as defined in item (1e) (e.g., the antibody or antigen-binding fragment thereof as described in the preceding items (e1) to (e3));
the capture antibody is the antibody or antigen-binding fragment thereof as defined in item (1c) (e.g., the antibody or antigen-binding fragment thereof as described in the preceding items (c1) to (c3)), and the detection antibody is the antibody or antigen-binding fragment thereof as defined in item (1f) (e.g., the antibody or antigen-binding fragment thereof as described in the preceding items (f1) to (f2));
the capture antibody is the antibody or antigen-binding fragment thereof as defined in item (1e) (e.g., the antibody or antigen-binding fragment thereof as described in the preceding items (e1) to (e3)), and the detection antibody is the antibody or antigen-binding fragment thereof as defined in item (1b) (e.g., the antibody or antigen-binding fragment thereof as described in the preceding items (b1) to (b3)).

In some embodiments, the capture antibody is selected from the group consisting of the following antibodies or antigen-binding fragments thereof as defined in preceding items (a1) to (a4); the detection antibody is selected from the group consisting of the following antibodies or antigen-binding fragments thereof as defined in preceding items (b1) to (b3), (d1) to (d3), (e1) to (e3) and (f1) to (f2).

In some embodiments, the capture antibody is selected from the group consisting of the following antibodies or antigen-binding fragments thereof as defined in preceding items (b1) to (b3); the detection antibody is selected from the group consisting of the following antibodies or antigen-binding fragments thereof as defined in preceding items (a1) to (a4), (c1) to (c3), (d1) to (d3), (e1) to (e3) and (g1) to (g2).

In some embodiments, the capture antibody is selected from the group consisting of the following antibodies or antigen-binding fragments thereof as defined in preceding items (c1) to (c3); the detection antibody is selected from the group consisting of the following antibodies or antigen-binding fragments thereof as defined in preceding items (b1) to (b3), (e1) to (e3) and (f1) to (f2).

In some embodiments, the capture antibody is selected from the group consisting of the following antibodies or antigen-binding fragments thereof as defined in preceding items (d1) to (d3); the detection antibody is selected from the group consisting of the following antibodies or antigen-binding fragments thereof as defined in preceding items (a1) to (a4), (b1) to (b3) and (f1) to (f2).

In some embodiments, the capture antibody is selected from the group consisting of the following antibodies or antigen-binding fragments thereof as defined in preceding items (e1) to (e3); the detection antibody is selected from the group consisting of the following antibodies or antigen-binding fragments thereof as defined in preceding items (b1) to (b3), (c1) to (c3) and (f1) to (f2).

In some embodiments, the method comprises:
(1) contacting the sample with the capture antibody to form an antibody-antigen complex;
(2) contacting the antibody-antigen complex with the detection antibody to form an antibody-antigen-antibody complex; and
(3) determining the amount of the antibody-antigen-antibody complex.

In some embodiments, the capture antibody is coated on the surface of a solid phase carrier.

In some embodiments, the solid phase carrier is selected from magnetic particles or microtiter plates (e.g., microplates or enzyme plates);

the detection antibody has a detectable label.

In some embodiments, the label is selected from the group consisting of fluorescein, chemiluminescence (e.g., acridinium esters), enzyme (e.g., horseradish peroxidase, alkaline phosphatase), radioisotope, biotin, and colloidal gold.

In some embodiments, the method is an ELISA immunoassay, such as a double-antibody sandwich ELISA immunoassay.

In some embodiments, the sample is from human, such as human body fluids (e.g., blood, serum, or plasma).

In some embodiments, the method comprises the following steps:
the capture antibody coated on the surface of the solid phase carrier is diluted with a capture antibody diluent to obtain a capture antibody reagent; preferably, the concentration of the capture antibody in the capture antibody reagent is 0.001% to 0.005%, such as 0.001% to 0.003%;
the detection antibody having a detectable label is diluted with a detection antibody diluent to obtain a detection antibody reagent; preferably, the mass concentration of the labeled detection antibody in the detection antibody reagent is 1 to 5 µg/mL, for example 1 to 3µg/mL;
1 to 20 µL of the sample is reacted with the capture antibody reagent and the detection antibody reagent, and is subjected to chemiluminescence immunoassay to obtain the presence or concentration of PINP and different forms of existence thereof in the sample;
preferably, the amount of the sample is 15 µL or less, preferably 10 µL or less, and more preferably 5 µL or less.

In some embodiments, the method further comprises an operation of using a PINP calibrator to perform calibration.

In some embodiments, the PINP calibrator comprises the amino acid sequence as set forth in SEQ ID NO: 43.

In some embodiments, the sample detection time of the method is less than 20 minutes, preferably less than 15 minutes.

In some embodiments, the detection range for tPINP in the sample is 0.03 ng/mL to 2000 ng/mL.

In another aspect, the present invention provides use of PINP as a detection quality control substance or a calibrator, and PINP has the amino acid sequence as set forth in SEQ ID NO: 43.

In some embodiments, the detection is an ELISA immunoassay, such as a double-antibody sandwich ELISA immunoassay.

In another aspect, the present invention provides use of the antibody or antigen-binding fragment thereof as described in any one of the preceding items in the manufacture of a kit for detecting the presence or level of tPINP (especially PINP) in a sample.

In another aspect, the present invention provides use of the composition or detection kit or method as described in any one of the preceding items for detecting the presence or level of tPINP (especially PINP) in a sample.

### Definition of Terms

In the present invention, unless otherwise indicated, scientific and technical terms used herein have meanings commonly understood by those skilled in the art. Moreover, the virological, biochemical, and immunological laboratory procedures used herein are routine procedures widely used in corresponding fields. Meanwhile, for better understanding of the present invention, definitions and explanations of relevant terms are provided below.

Among the organic components of bone matrix, collagen type I has a content of exceeding 90%, fibroblasts and osteoblasts first synthesize procollagen I, and then the later forms collagen type I. Procollagen I has extended peptide chains at its amino terminus (N-terminal) and carboxyl terminus (C-terminal). These extended peptide chains (propeptides) are cleaved off by specific proteases during the conversion of procollagen to collagen. When mature collagen is formed, it would be deposited in the bone matrix. As used herein, the term "PINP" refers specifically to the procollagen I N-terminal propeptide. PINP reflects the deposition of collagen type I and therefore serves as a bone formation marker. During the formation of collagen type I, PINP is released into the extracellular space and eventually enters the bloodstream. PINP is in the trimeric form (converted from trimeric collagen), but quickly turns into a monomeric form under thermal degradation. The kit and detection method of the present invention can be used to detect all PINP forms in blood, namely tPINP, including monomeric form and trimeric form thereof.

As used herein, the term "specific binding" refers to a non-random binding reaction between two molecules (i.e., a binding molecule and a target molecule), such as reaction between an antibody and a target antigen thereof. The binding affinity between two molecules can be described by the K_{D} value. The K_{D} value refers to a dissociation constant obtained from the ratio of kd (dissociation rate of specific binding molecule-target molecule interaction; also known as koff) to ka (association rate of specific binding molecule-target molecule interaction; also known as kon), or refers to kd/ka that is expressed as molar concentration (M). The smaller the K_{D} value, the tighter the binding between the two molecules and the higher the affinity. In certain embodiments, an antibody that specifically binds to an antigen (or an antibody that is specific for an antigen) refers to an antibody that binds to the antigen with an affinity (K_{D}) of less than about 10⁻⁵ M, such as less than about 10⁻⁶ M, 10⁻⁷ M, 10⁻⁸ M, 10⁻⁹ M, or 10⁻¹⁰ M or less. The K_{D} value can be determined by methods well known in the art, such as by surface plasmon resonance (SPR) in a BIACORE instrument.

As used herein, the term "immunological assay" refers to an assay that utilizes specific antigen-antibody interaction/binding affinity, and is generally used to detect the presence or level of a specific antigen or antibody in a sample. Such immunological assays are well known to those skilled in the art, and include, but are not limited to, enzyme immunoassay (EIA), chemiluminescence immunoassay (CLIA), radioimmunoassay (RIA), fluorescent immunoassay (FIA), Western blotting, immunoturbidimetry, surface plasmon resonance, etc. For a detailed description of immunological assays, see, for example, Fundamental Immunology, Ch. 7 Paul, W., ed., 2nd edition, Raven Press, N.Y. (1989).

As used herein, the terms "antibody" and "monoclonal antibody" are used interchangeably and refer to an immunoglobulin molecule typically consisting of two pairs of polypeptide chains (each pair having a light chain (LC) and a heavy chain (HC)). Antibody light chains can be classified into κ (kappa) and λ (lambda) light chains. Heavy chains can be classified as µ, δ, γ, α, or ε, and the isotypes of antibody are accordingly defined as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin to host tissues or factors, including the various cells of immune system (e.g., effector cells) and the first component of classical complement system (C1q). The VH and VL regions can also be subdivided into regions of high variability called complementarity determining regions (CDRs), interspersed therewith more conservative regions called framework regions (FRs). Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (VH and VL) of each heavy chain/light chain pair respectively form antigen-binding site. The assignment of amino acids to regions or domains can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity determining region" or "CDR" refers to the amino acid residues in a variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (for example, see Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia, or IMGT numbering systems.

As used herein, the term "framework region" or "FR" residues refers to those amino acid residues in an antibody variable region other than the CDR residues as defined above.

The term "antibody" is not limited to any particular method of producing the antibody. It includes, for example, recombinant antibodies, monoclonal antibodies, and polyclonal antibodies. The antibody may be of different isotypes, for example, IgG (e.g., IgG1, IgG2, IgG3 or IgG4 subtype), IgA1, IgA2, IgD, IgE or IgM antibody.

As used herein, the term "antigen-binding fragment" of antibody refers to a polypeptide comprising a fragment of a full-length antibody that retains the ability to specifically bind to the same antigen that the full-length antibody binds, and/or competes with the full-length antibody for specific binding to the antigen, which is also called as "antigen-binding moiety." See generally, Fundamental Immunology, Ch. 7 (Paul, W., ed., 2nd ed., Raven Press, N.Y. (1989), which is incorporated herein by reference in its entirety for all purposes. The antigen-binding fragment of antibody can be generated by recombinant DNA technology or by enzymatic or chemical cleavage of an intact antibody. Non-limiting examples of antigen-binding fragments include Fab, Fab', F(ab')₂, Fd, Fv, complementarity determining region (CDR) fragments, scFv, diabody, single domain antibody, chimeric antibody, linear antibody, nanobody (its technology is from Domantis) and such polypeptides, which contain at least a portion of antibody that is sufficient to confer the specific antigen-binding ability to the polypeptide. Engineered antibody variants are reviewed by Holliger et al., 2005; Nat Biotechnol, 23: 1126-1136.

As used herein, the term "Fd" refers to an antibody fragment consisting of VH and CH1 domains; the term "dAb fragment" refers to an antibody fragment consisting of VH domain (Ward et al., Nature 341:544 546 ( 1989)); the term "Fab fragment" refers to an antibody fragment consisting of VL, VH, CL and CH1 domains; the term "F(ab')₂ fragment" refers to an antibody fragment consisting of two Fab fragments connected by a disulfide bridge on the hinge region; the term "Fab' fragment" refers to a fragment obtained by reducing the disulfide bond connecting the two heavy chain fragments in the F(ab')₂ fragment, consisting of a complete light chain and an Fd fragment (consisting of VH and CH1 domains) of heavy chain.

As used herein, the term "Fv" refers to an antibody fragment consisting of the VL and VH domains of a single arm of an antibody. Fv fragments are generally considered to be the smallest antibody fragments that can form a complete antigen-binding site. It is generally believed that six CDRs confer the antigen-binding specificity to the antibody. However, even a variable region (e.g., an Fd fragment, which contains only three antigen-specific CDRs) can recognize and bind to the antigen, although its affinity may be lower than that of the intact binding site.

As used herein, the term "scFv" refers to a single polypeptide chain comprising VL and VH domains, in which the VL and VH are connected by a linker (see, for example, Bird et al., Science 242:423 -426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH₂-VL-linker-VH-COOH or NH₂-VH-linker-VL-COOH. Suitable prior art linkers consist of repeated GGGGS amino acid sequences or variants thereof. For example, a linker having the amino acid sequence (GGGGS)₄ can be used, but variants thereof can also be used (Holliger et al. (1993), Proc. Natl. Acad. Sci. USA 90: 6444-6448). Other linkers useful in the present invention are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between VH and VL of scFv.

As used herein, the term "diabody" means that its VH and VL domains are expressed on a single polypeptide chain, but using a linker that is too short to allow pairing between the two domains of the same chain, and this forces the domains to pair with the complementary domains of another chain and creates two antigen-binding sites (see, for example, Holliger P. et al., Proc. Natl. Acad. Sci. USA 90:6444-6448 (1993), and Poljak R. J. et al., Structure 2:1121-1123 (1994)).

As used herein, the term "single-domain antibody (sdAb)" has the meaning commonly understood by those skilled in the art, and refers to an antibody fragment consisting of a single monomeric variable antibody domain (e.g., a single heavy chain variable), which retains the ability to specifically bind to the same antigen that the full-length antibody binds. Single-domain antibody is also called nanobody.

Each of the above antibody fragments retains the ability to specifically bind to the same antigen that the full-length antibody binds, and/or competes with the full-length antibody for specific binding to the antigen.

Antigen-binding fragments of antibody (e.g., the above-described antibody fragments) can be obtained from a given antibody (e.g., the antibody provided by the present invention) using conventional techniques known to those skilled in the art (e.g., recombinant DNA technology or enzymatic or chemical fragmentation methods), and the antigen-binding fragments of antibody are screened for specificity in the same manner as for the intact antibody.

As used herein, when the term "antibody" is mentioned, it includes not only an intact antibody but also antigen-binding fragments of the antibody, unless the context clearly indicates otherwise.

As used herein, the term "chimeric antibody" refers to an antibody in which a part of the light chain and/or heavy chain is derived from one antibody (which may be derived from a specific species or belong to a specific antibody class or subclass), and the other part of the light chain or/and heavy chain is derived from another antibody (which may be derived from the same or different species or belong to the same or different antibody class or subclass), but nevertheless, it still retains the binding activity to a target antigen (U.S.P 4,816,567 to Cabilly et al.; Morrison et al., Proc. Natl. Acad. Sci. USA, 81:6851 6855 (1984)). For example, the term "chimeric antibody" may include such an antibody (e.g., human-mouse chimeric antibody) in which the heavy and light chain variable regions of the antibody are derived from a first antibody (e.g., a murine antibody), while the heavy and light chain variable regions of the antibody are derived from a secondary antibody (e.g., a human antibody).

As used herein, the term "humanized antibody" refers to a non-human antibody that has been genetically engineered and its amino acid sequence has been modified to increase sequence homology to that of a human antibody. Generally speaking, all or part of the CDR regions of a humanized antibody come from a non-human antibody (donor antibody), and all or part of the non-CDR regions (for example, variable region FR and/or constant regions) come from a human immunoglobulin (receptor antibody). The humanized antibody generally retains the expected properties of the donor antibody, including but not limited to, antigen specificity, affinity, reactivity, etc. The donor antibody may be a mouse, rat, rabbit, or non-human primate (e.g., cynomolgus monkey) antibody with desired properties (e.g., antigen specificity, affinity, reactivity, etc.).

The chimeric antibody or humanized antibody of the present invention can be prepared according to the sequence of the mouse monoclonal antibody prepared above. The DNA encoding the heavy and light chains can be obtained from a mouse hybridoma of interest and engineered to contain non-murine (e.g., human) immunoglobulin sequences using standard molecular biology techniques.

To prepare the chimeric antibody, a murine immunoglobulin variable region can be ligated to a human immunoglobulin constant region using a method known in the art. For example, the DNA encoding VH is operably ligated to another DNA molecule encoding heavy chain constant region to obtain a full-length heavy chain gene. The sequences of human heavy chain constant region genes are known in the art (see, for example, Kabat, E.A. et al. (1991) Sequences of Proteins of Immunological Interest, Fifth Edition, U.S. Department of Health and Human Services, NIH Publication No. 91-3242), and DNA fragments containing these regions can be obtained by standard PCR amplification. The heavy chain constant region may be an IgGl, IgG2, IgG3, IgG4, IgA, IgE, IgM or IgD constant region, but is generally preferred to be an IgGl or IgG4 constant region. For example, the DNA encoding VL is operably ligated to another DNA molecule encoding light chain constant region CL to obtain a full-length light chain gene (as well as an Fab light chain gene). The sequences of human light chain constant region genes are known in the art (see, for example, Kabat, E.A. et al. (1991) Sequences of Proteins of Immunological Interest, Fifth Edition, U.S. Department of Health and Human Services, NIH Publication No. 91-3242), and DNA fragments containing these regions can be obtained by standard PCR amplification. The light chain constant region may be a κ or λ constant region, but a κ constant region is generally preferred.

To prepare the humanized antibody, a murine CDR region can be inserted into a human framework sequence using a method known in the art (see, Winter, U.S. Patent Nos. 5,225,539; Queen et al., U.S. Patent Nos. 5,530,101; 5,585,089; 5,693,762 and 6,180,370; and Lo, Benny, K.C., editor, in Antibody Engineering: Methods and Protocols, volume 248, Humana Press, New Jersey, 2004).

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC) or P1-derived artificial chromosome (PAC); phages such as lambda phage or M13 phage and animal viruses, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector can contain a variety of expression control elements, including, but not limited to, promoter sequence, transcription initiation sequence, enhancer sequence, selection element and reporter gene. In addition, the vector may also contain an origin of replication site.

As used herein, the term "host cell" refers to a cell that can be used to introduce a vector, which includes, but is not limited to, prokaryotic cell such as *E. coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "identity" is used to refer to the match of sequences between two polypeptides or between two nucleic acids. When a position in both sequences being compared is occupied by the same base or amino acid monomer subunit (e.g., a position in each of two DNA molecules is occupied by adenine, or a position in each of two polypeptides is occupied by lysine), then the molecules are identical at that position. "Percent identity" between two sequences is a function of the number of matching positions shared by the two sequences divided by the number of positions compared × 100. For example, if 6 out of 10 positions of two sequences match, then the two sequences have an identity of 60%. For example, the DNA sequences CTGACT and CAGGTT have an identity of 50% (3 out of 6 positions matching). Typically, comparisons are made when two sequences are aligned to yield maximum identity. Such alignment can be accomplished using, for example, the method of Needleman et al. (1970) J. Mol. Biol. 48:443-453, which can be conveniently performed by a computer program such as the Align program (DNAstar, Inc.). The percent identity between two amino acid sequences can also be determined by using the algorithm of E. Meyers and W. Miller (Comput. Appl Biosci., 4:11-17 (1988)) that has been integrated into the ALIGN program (version 2.0), and using the PAM120 weight residue table, a gap length penalty of 12 and a gap penalty of 4. Alternatively, the percent identity between two amino acid sequences can be determined by using the algorithm of Needleman and Wunsch (J MoI Biol. 48:444-453 (1970)) that had been integrated into the GAP program in the GCG software package (available on www.gcg.com), and using the Blossum 62 matrix or PAM250 matrix, a gap weight of 16, 14, 12, 10, 8, 6 or 4 and a length weight of 1, 2, 3, 4, 5 or 6.

As used herein, the term "conservative substitution" refers to an amino acid substitution that does not adversely affect or alter the expected properties of protein/polypeptide comprising the amino acid sequence. For example, a conservative substitution can be introduced by standard techniques known in the art, such as site-directed mutagenesis and PCR-mediated mutagenesis. Conservative amino acid substitutions include those in which an amino acid residue is replaced with an amino acid residue having a similar side chain, e.g., substitution with a residue physically or functionally similar to the corresponding amino acid residue (e.g., having similar size, shape, charge, chemical properties including ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine amino acids, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which is incorporated herein by reference).

The twenty conventional amino acids involved herein have been written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference.

In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

As used herein, the term "subject" includes, but is not limited to, various animals, particularly mammals, such as human.

In the present application, unless otherwise indicated, "%" refers to mass percentage.

### Brief Description of the Drawings

Fig. 1 shows the binding curves of the antibodies obtained from biolayer interferometry measurement, wherein A, B, C, D, E, F and G correspond to PINP01, hPINP02, hPINP03, hPINP04, hPINP05, hPINP06 and hPINP07, respectively.
Fig. 2 shows the detection range and linearity of the kit of Example 6.
Fig. 3 shows the detection range and linearity of the kit of Example 7.
Fig. 4 shows the detection range and linearity of the kit of Example 8.
Fig. 5 shows the detection range and linearity of the kit of Example 9.
Fig. 6 shows the detection range and linearity of the kit of Example 10.
Fig. 7 shows the detection range and linearity of the kit of Example 11.

### Specific Models for Carrying Out the present Invention

The embodiments of the present invention will be described in detail below with reference to examples, but those skilled in the art will understand that the following examples are only used to illustrate the present invention and should not be regarded as limiting the scope of the present invention. If the specific conditions were not specified in the examples, the conventional conditions or the conditions recommended by the manufacturer should be applied. If the manufacturer of the reagents or instruments used was not indicated, they were all conventional products that could be purchased commercially.

### Example 1: Generation of monoclonal antibodies specific to human Procollagen I N-Terminal Propeptide

Recombinant A1-PINP was used for immunization of mice and rats, wherein sequence of recombinant A1-PINP (HyTest Cat# 8PIN7) corresponds to region 23-161 (N-terminal propeptide) of α1 chain of human procollagen type I (UniProt ID P02452). Anti-A1-PINP murine antibodies PINP01, PINP02, PINP03, PINP04, PINP05, PINP06and PINP07 were obtained through hybridoma screening, wherein PINP01 was a mouse antibody, and the rest were rat antibodies. These 7 antibodies were sequenced, and the results were shown in Tables 1 and 2.

**Table 1: Sequencing results for heavy chains of the antibodies**

| Antibody name | | Heavy chain | | |
|---|---|---|---|---|
| | | CDR1 | CDR2 | CDR3 |
| PINP01 | nucleotide sequence | AGCTACTGGA TGAAC | ATGATTCATCCTTCCGATAG TGAAACTAGGTTAAATCAGA AGTTCAAGGAC | ACCCACATCTAC TATGTCTTTGACT AC |
| | amino acid sequence | SYWMN | MIHPSDSETRLNQKFKD | THIYYVFDY |
| | Numbering system | Kabat | Kabat | Kabat |
| PINP02 | nucleotide sequence | AATGCTGCCA TGTAC | CGCATAAGAACTAAACCTAA TAATTATGCAACATATTATG CTGATTCAGTGAAAGGC | ACGACTACGGAT ATGGGTCCTATG GGGTAC |
| | amino acid sequence | NAAMY | RIRTKPNNYATYYADSVKG | TTTDMGPMGY |
| | Numbering system | Kabat | Kabat | Kabat |
| PINP03 | nucleotide sequence | AACAATGGTG TAAGC | GCAATGTCAAGTGGTGGAA ACACATATTATAATTCAGGT CTCAAATCC | CACGACGCGGGG CTAATTTAC |
| | amino acid sequence | NNGVS | AMSSGGNTYYNSGLKS | HDAGLIY |
| | Numbering system | Kabat | Kabat | Kabat |
| PINP04 | nucleotide sequence | GACTATTACA TGGCC | ACCATTAGTTATGATGGTAG TGGCACTCACTATCGAGACT CCGTGAAGGGC | CGAAGGGGTTAC GGCTATTACTAT GTTATGGATGCC |
| | amino acid sequence | DYYMA | TISYDGSGTHYRDSVKG | RRGYGYYYVMD A |
| | Numbering system | Kabat | Kabat | Kabat |
| PINP05 | nucleotide sequence | AATGCTGCCA TGTAC | CGCATAAGAACTAAAGTTAA TAATTATGCAACATATTATG | GCGGGCTATTAT GATGGTACTTATT |
| | | | CTGATTCAGTGAGAGGC | ATTACGAGGGCG ACTACTTTGATTA C |
| | amino acid sequence | NAAMY | RIRTKVNNYATYYADSVRG | AGYYDGTYYYEG DYFDY |
| | Numbering system | Kabat | Kabat | Kabat |
| PINP06 | nucleotide sequence | AATGCTGCCA TGTAC | CGCATAAGAACTAAACCTAA GAGACTCGCAAC TGATTATGCAACATATTACA TTGGATTGGTTTG CTGATTCAGTGAAAGGC CTTAC | GAGACTCGCAAC TTGGATTGGTTTG CTTAC |
| | amino acid sequence | NAAMY | RIRTKPNDYATYYTDSVKG | ETRNLDWFAY |
| | Numbering system | Kabat | Kabat | Kabat |
| PINP07 | nucleotide sequence | GGTTCCAGTG TGGGCGTGGG C | ACCATTGGGTGGGAGGATGT TAAGCACTACAATCCATCTC TGAAGAGC | TCCTGGGAGAGG TTTGATTAC |
| | amino acid sequence | GSSVGVG | TIGWEDVKHYNPSLKS | SWERFDY |
| | Numbering system | Kabat | Kabat | Kabat |

**Table 2: Sequencing results of light chains for the antiboides**

| Antibody name | | Light chain | | |
|---|---|---|---|---|
| | | CDR1 | CDR2 | CDR3 |
| PINP01 (Kappa) | nucleotide sequence | AAATCCAGTCAGAGTCTG CTCAACAGTAGAACCCG AAAGAACTACTTGGCT | TGGGCATCCA CTAGGGAATC T | AAGCAATCTTA TAATCTGTACA CG |
| | amino acid sequence | KSSQSLLNSRTRKNYLA | WASTRES | KQSYNLYT |
| | Numbering system | Kabat | Kabat | Kabat |
| PINP02 (Kappa) | nucleotide sequence | CGGGCAAGTCAAGACAT TGGAAATTATTTAAGA | GGTGAAACCA ACTTGGCAAA T | CTGCAGCATTA TGATTATCCTCT CACG |
| | amino acid sequence | RASQDIGNYLR | GETNLAN | LQHYDYPLT |
| | Numbering system | Kabat | Kabat | Kabat |
| PINP03 (Kappa) | nucleotide sequence | AAGGCCAGTCAGAATGT GGGTACTAATGTAGAC | GGGGCATCCA ACCGGTACAC T | CTACAGTATAA CGACACTCCGT GGACG |
| | amino acid sequence | KASQNVGTNVD | GASNRYT | LQYNDTPWT |
| | Numbering system | Kabat | Kabat | Kabat |
| PINP04 (Kappa) | nucleotide sequence | AAGGCCAGTCAAAATGT GGGTTCTAATGTAGAC | AAAGCATCCA ACCTGTACAC G | ATGCAGTCTGG CTCCTATCCTCC AACG |
| | amino acid sequence | KASQNVGSNVD | KASNLYT | MQSGSYPPT |
| | Numbering system | Kabat | Kabat | Kabat |
| PINP05 (Kappa) | nucleotide sequence | AGGGCCAGCCAAAGTGT CAGTATAACTGGATATAG TCTTATGCAC | CGTACATCCG ACCTAGCATCT | CAGCAGAGTAG GGAGTCTCCTC CGACG |
| | amino acid sequence | RASQSVSITGYSLMH | RTSDLAS | QQSRESPPT |
| | Numbering system | Kabat | Kabat | Kabat |
| PINP06 (Kappa) | nucleotide sequence | AGATCTAGTAAGAGTCCG CGACATAGTAATGGCATC ACTTACGTGTAT | CGGATGTCCA ACCTTGCCTCA | GGACAGGTTCT AGAAAATCTTC CGACG |
| | amino acid sequence | RSSKSPRHSNGITYVY | RMSNLAS | GQVLENLPT |
| | Numbering system | Kabat | Kabat | Kabat |
| PINP07 (Lambda) | nucleotide sequence | TCTGGAGATGAGTTACCA AAAAGATATGCTTAT | AAAGATAGTG AGCGGCCCTC A | CACTCGACATA TAGTGATGATA AACTCCCTGTT |
| | amino acid sequence | SGDELPKRYAY | KDSERPS | HSTYSDDKLPV |
| | Numbering system | Kabat | Kabat | Kabat |

### Example 2: Production and affinity purification of antibodies

The heavy chain variable region of murine antibodies PINP02, PINP03, PINP04, PINP05, PINP06, and PINP07 were respectively combined with the human IgG1 heavy chain constant region, and the light chain variable region of the above antibodies were respectively combined with the human λ light chain constant region to develop genetic constructs of 6 recombinant antibodies. The genetic constructs were respectively expressed in mammalian cells to obtain recombinant antibodies hPINP02, hPINP03, hPINP04, hPINP05, hPINP06, hPINP07, and purified.

The expression vectors containing the gene sequences of the above-mentioned recombinant antibodies could be obtained through bacterial culture and plasmid purification. The expression vectors were transfected into mammalian cell line Expi293F.

For example, through methods such as homologous recombination or restriction enzyme digestion, the light chain and heavy chain gene sequences were respectively transferred to the open reading frame (ORF) of expression vector, and finally "promoter-antibody light chain gene-terminator" and "promoter-antibody heavy chain gene-terminator" structures were formed on the two expression vectors respectively; then, the two expression vectors (antibody light chain expression vector and heavy chain expression vector) were simultaneously transferred by biological, physical, and chemical methods into the mammalian cell line Expi293F and expressed; and finally, the two light chains and the two heavy chains were reassembled into a intact antibody in the cells and secreted into the culture supernatant. Alternatively, the expression vector was modified so that one expression vector contained two open reading frames, and then the light chain and heavy chain gene sequences were inserted into the two open reading frames (ORFs) of the expression vector through homologous recombination or restriction enzyme digestion to form "promoter-antibody light chain gene-terminator-vector sequence-promoter-antibody heavy chain gene-terminator" or "promoter-antibody heavy chain gene-terminator-vector sequence-promoter-antibody light chain gene-terminator" structure; then the expression vector containing both the gene sequences of antibody light chain and heavy chain was transferred by biological, physical, and chemical methods into the mammalian cell line Expi293F and expressed; and finally, the two light chains and the two heavy chains were reassembled into an intact antibody in the cells and secreted into the culture supernatant.

Antibodies (PINP01, hPINP02, hPINP03, hPINP04, hPINP05, hPINP06, hPINP07) were purified from conditioned culture media by using protein A affinity chromatography. The packing material of affinity chromatography was from GE health care Life Sciences (Piscataway, NJ). Purification was carried out according to manufacturer's instructions. The purified monoclonal antibodies were stored as suspensions in 50% ammonium sulfate at 4°C.

### Deposit information

PINP01: The hybridoma cell line producing PINP01 was deposited with VKPM on February 21, 2024, having Accession Number H-214.

hPINP02: The plasmid comprising the insert encoding VH amino acid sequence of hPINP02 was deposited with VKPM on March 25, 2024, having Accession Number B-14744, the plasmid comprising the insert encoding VL amino acid sequence of hPINP02 was deposited with VKPM on March 25, 2024, having Accession Number B-14745.

hPINP03: The plasmid comprising the insert encoding VH amino acid sequence of hPINP03 was deposited with VKPM on March 25, 2024, having Accession Number B-14746, the plasmid comprising the insert encoding VL amino acid sequence of hPINP03 was deposited with VKPM on March 25, 2024, having Accession Number B-14747.

hPINP04: The plasmid comprising the insert encoding VH amino acid sequence of hPINP04 was deposited with VKPM on March 25, 2024, having Accession Number B-14748, the plasmid comprising the insert encoding VL amino acid sequence of hPINP04 was deposited with VKPM on March 25, 2024, having Accession Number B-14749.

hPINP05: The plasmid comprising the insert encoding VH amino acid sequence of hPINP05 was deposited with VKPM on March 25, 2024, having Accession Number B-14750, the plasmid comprising the insert encoding VL amino acid sequence of hPINP05 was deposited with VKPM on March 25, 2024, having Accession Number B-14751.

hPINP06: The plasmid comprising the insert encoding VH amino acid sequence of hPINP06 and the insert encoding VL amino acid sequence of hPINP06 was deposited with VKPM on March 25, 2024, having Accession Number B-14752.

hPINP07: The plasmid comprising the insert encoding VH amino acid sequence of hPINP07 and the insert encoding VL amino acid sequence of hPINP07 was deposited with VKPM on March 25, 2024, having Accession Number B-14753.

### Example 3: Measurement of the affinity of the antibodies

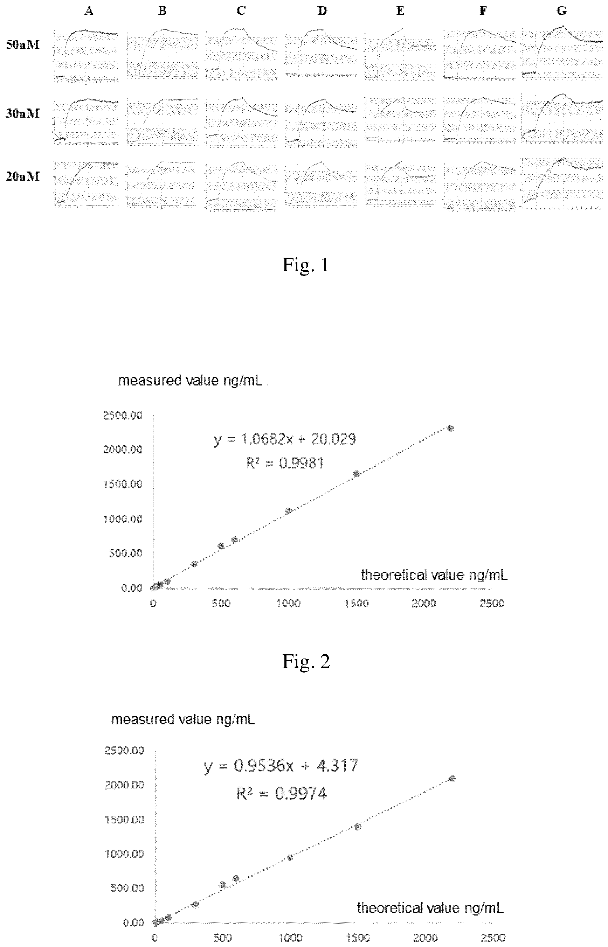

Biolayer interferometry measurement was performed according to the following protocol to determine the affinity of the antibodies for A1-PINP, the results were specifically shown in Fig. 1 and Table 3.

Antibody (2 µg/ml) in PBS solution was bound to the sensor, and the sensor was blocked, and then the antigen (A1-PINP) at concentrations of 20, 30, and 50 nM was reacted with the antibody on the sensor. The complete scheme of the experiment was as follows: sensor hydration → antibody binding to the sensor → washing of the sensor → blocking of the sensor → association (separately for each concentration of tested antibody) → dissociation → regeneration of the sensor.

**Table 3: Affinity measurement results of the monoclonal antibodies**

| Antibody | Ka (1/Ms) | Kd (1/s) | KD (M) |
|---|---|---|---|
| PINP01 | 1.59E+05 | 4.93E-05 | 3.59E-10 |
| hPINP02 | 3.83E+04 | 1.37E-04 | 3.6E-09 |
| hPINP03 | 1.53E+05 | 5.04E-04 | 3.29E-09 |
| hPINP04 | 1.47E+05 | 3.1E-04 | 2.14E-09 |
| hPINP05 | 1.61E+05 | 1.97E-04 | 1.54E-09 |
| hPINP06 | 1.18E+05 | 1.37E-04 | 1.2E-09 |
| hPINP07 | 1.03E+05 | 1.89E-04 | 2.02E-09 |

### Example 4: Development of chemiluminescent "sandwich" immunoassay

All mAbs were tested in different pair combinations using sandwich immunoassay to find combinations with the best properties.

To perform chemiluminescent sandwich particle-immunoassay, the detection mAb was labeled with alkaline phosphatase (Cat. No. 03137031103, Roche Custom Biotech), and the capture mAb were labeled with biotin (Cat. No. PG82075, Thermo). The capture antibody was incubated with Streptavidin-labeled magnetic beads (MyOne T1 Dynabeads, Streptavidin PMP Hydrophobic Particles, 100 mkm, Cat# 35604D, Thermo), and the incubated magnetic beads were diluted with a solution containing 50 mM Tris-HCl, pH 7.4, 150 mM NaCl, 2% BSA, 0.2% Tween-20, 0.05% ProClin 300 and 0.09% NaN₃ to obtain a magnetic bead suspension with magnetic beads concentration of 0.25 mg/ml. The detection antibody-alkaline phosphatase conjugate was diluted in a solution containing 50 mM MES, pH 6, 0.9% NaCl, 1% BSA, 0.05% Tween-20, 0.048% ProClin 300, 2 mM MgCl₂ and 0.1 mM ZnCl₂ to obtain 0.5 µg/ml detection antibody-alkaline phosphatase conjugate solution. A1-PINP was used as calibrator for the immunoassay. Serial dilutions of A1-PINP were prepared using a solution containing 50 mM MES, pH 6, 0.9% NaCl, 1% BSA, 0.05% Tween-20, 0.048% ProClin 300, 2 mM MgCl₂, and 0.1 mM ZnCl₂. 20 µL of A1-PINP diluent was mixed with 50 µL of the magnetic bead suspension and 50 µL of the detection antibody-alkaline phosphatase conjugate solution. The one-step immunoassay was carried out in automatic analyzer Mindray CL-6000i according to the instructions, and the substrate solutions provided by the analyzer supplier were used. Chemiluminescence was expressed in relative light units (RLU). The above method was able to detect both trimeric and monomeric forms of recombinant human procollagen I N-terminal propeptide.

### Antibody pairs limit of detection (LOD) determination

LOB and LOD determination were performed according to Clinical and Laboratory Standards Institute (CLSI) recommendations (Evaluation of Detection Capability for Clinical Laboratory Measurement Procedures; Approved Guidelines - Second Edition; EP17-A, Volume 24, Issue 34). 20 replicates of a zero analyte (50 mM MES, pH6, 0.9% NaCl, 1% BSA, 0.05% Tween-20, 0.048% ProClin 300, 2 mM MgCl₂, 0.1 mM ZnCl₂) were assayed in corresponding chemiluminescent sandwich immunoassay.

LOB (limit of blank) and LOD were calculated according to the following general formulas:
LOB = 1.64 *mean;
LOD = LOB+1.65*SD

**Table 4: LOD for antibody pairs in CLIA**

| | Capture antibody | Detection antibody | LOD, ng/ml |
|---|---|---|---|
| Combination 1 | hPINP02 | PINP01 | 0.15 |
| Combination 2 | hPINP04 | PINP01 | 0.22 |
| Combination 3 | PINP01 | hPINP06 | 0.11 |
| Combination 4 | hPINP03 | hPINP06 | 0.25 |
| Combination 5 | hPINP04 | hPINP06 | 0.30 |
| Combination 6 | hPINP05 | hPINP06 | 0.26 |
| Combination 7 | hPINP02 | hPINP07 | 0.20 |
| Combination 8 | PINP01 | hPINP02 | 0.15 |
| Combination 9 | hPINP03 | hPINP02 | 0.14 |
| Combination 10 | hPINP04 | hPINP02 | 0.26 |
| Combination 11 | hPINP05 | hPINP02 | 0.30 |
| Combination 12 | hPINP02 | hPINP03 | 0.21 |
| Combination 13 | hPINP05 | hPINP03 | 0.19 |
| Combination 14 | PINP01 | hPINP04 | 0.28 |
| Combination 15 | hPINP02 | hPINP04 | 0.18 |
| Combination 16 | PINP01 | hPINP05 | 0.40 |
| Combination 17 | hPINP02 | hPINP05 | 0.36 |
| Combination 18 | hPINP03 | hPINP05 | 0.18 |

### Example 5: Sequencing of antigen used for quality control or as calibrator in PINP kits

The A1-PINP coding sequence (SEQ ID NO: 43) was available from public database and corresponded to the 23-161 region of human collagen α-1(I) chain (Uniprot P02452, COL1A1 - collagen α-1(I) chain - Homo sapiens (Human) | UniProtKB | UniProt). Coding nucleotide sequence was synthesized in Genscript and subcloned into pcDNA3.4 expression vector using standard cloning techniques. Gene construct contained H6 affinity tag at the C-terminus separated from A1-PINP sequence by GGGSGGGSGGGS linker. Endotoxin-free plasmid DNA was purified using Zymo Research DNA plasmid purification Kit. The Expi293F cell line was transfected with linear PEI and culture medium collected at day 7. Cell-free medium was subjected to further metal-chelate chromatography and purified to homogeneity.
SEQ ID NO: 43

MFSFVDLRLLLLLAATALLTHGQEEGQVEGQDEDIPPITCVQNGLRYHDRDVWKPEPCRICV
CDNGKVLCDDVICDETKNCPGAEVPEGECCPVCPDGSESPTDQETTGVEGPKGDTGPRGPRGPA
GPPGRDGIPGQPGLPGPPGPPGPPGPPGLGGNFAPGGGSGGGSGGGSHHHHHH

Signal peptide was underlined.

### Example 6: Preparation of tPINP kit and calibrator, and detection of tPINP

### Step 1: Preparation of R1 reagent

Specific PINP antibody (PINP01) was mixed with superparamagnetic particles in TBS buffer, allowed to react sufficiently, then placed on a magnetic separator until the supernatant was no longer turbid, then the supernatant was discarded. The superparamagnetic particles coated with PINP antibody were kept and washed three times with TBS buffer. Finally, the superparamagnetic particles coated with PINP antibody were dissolved with R1 diluent, and the final content of superparamagnetic particles was 0.07% and the content of PINP antibody was 0.002 %, wherein R1 diluent was composed of: 50 mM Tris, 0.9% NaCl, 0.048% Proclin300, 0.1% Tween-20, 1% BSA, pH 8.0, and the R1 reagent was finally obtained and stored at 2 to 8 °C.

### Step 2: Preparation of R2 reagent

Another specific PINP antibody (hPINP04) was coupled to alkaline phosphatase, wherein the molar ratio of the obtained conjugate to antibody was 15:1, and then the conjugate was dissolved with R2 diluent, and the final concentration of the conjugate was 2 µg/mL, wherein R2 diluent was composed of: 50 mM Tris, 0.9% NaCl, 0.048% Proclin300, 0.1% Tween-20, 1% BSA, pH 8.0, and finally the R2 reagent was obtained and stored at 2 to 8 °C.

### Step 3: Preparation of PINP calibrators

The PINP antigen A1-PINP was diluted to reach concentrations at, such as 0 ng/mL, 5 ng/mL, 10 ng/mL, 20 ng/mL, 50 ng/mL, 100 ng/mL, 300 ng/mL, 500 ng/mL, 1000 ng/mL, 1600 ng/mL, and 2200 ng/mL, etc. respectively, thereby obtaining PINP calibrators.

### Step 4: Measurement procedure of tPINP Kit

① The tPINP kit (including R1 reagent and R2 reagent) was loaded into the Mindray CL series fully automatic chemiluminescence immunoassay analyzer;
② The system required a sample volume of 5 µL for each measurement, and the total measurement time for each report was 17.5 minutes;

The specific sample addition process of the detection system was as follows: the fully automatic chemiluminescence immunoassay analyzer was used, 5 µL of sample, 50 µL of R1 reagent and 50 µL of R2 reagent were added to reaction tubes, respectively. After incubation, the PINP in the sample bound to the anti-PINP antibody coated on the magnetic beads, and at the same time, the anti-PINP antibody-alkaline phosphatase conjugate bound to another site of the PINP in the sample to form a sandwich complex. After the reaction was complete, the magnetic beads were attracted by a magnetic field, and the unbound substances were washed away.

Then a reaction substrate or excitation solution was added into the reaction tube to generate a light signal, and the number of photons generated was measured. The number of photons generated was proportional to the concentration of PINP in the sample.

③ The corresponding PINP calibrators were used to perform the calibration assays. Through the calibration data, the system software used a weighted four-parameter logarithmic curve (4PLC) mathematical method to fit the luminescence signal and concentration. The final results were given in the concentration form of ng/mL.

### Example 7: Preparation of tPINP kit and calibrator, and detection of tPINP

### Step 1: Preparation of R1 reagent

The specific PINP antibody (hPINP03) was mixed with superparamagnetic particles in TBS buffer, allowed to react sufficiently, then placed on a magnetic separator until the supernatant was no longer turbid, then the supernatant was discarded. The superparamagnetic particles coated with PINP antibody were kept and washed three times with TBS buffer. Finally, the superparamagnetic particles coated with PINP antibody were dissolved with R1 diluent, and the final content of superparamagnetic particles was 0.07% and the content of PINP antibody was 0.002 %, wherein R1 diluent was composed of: 50 mM Tris, 0.9% NaCl, 0.048% Proclin300, 0.1% Tween-20, 1% BSA, pH 8.0, and the R1 reagent was finally obtained and stored at 2 to 8 °C..

### Step 2: Preparation of R2 reagent

Another specific PINP antibody (hPINP06) was coupled to alkaline phosphatase, wherein the molar ratio of the obtained conjugate to antibody was 15:1, and then the conjugate was dissolved with R2 diluent, and the final concentration of the conjugate was 2 µg/mL, wherein R2 diluent was composed of: 50 mM Tris, 0.9% NaCl, 0.048% Proclin300, 0.1% Tween-20, 1% BSA, pH 8.0, and finally the R2 reagent was obtained and stored at 2 to 8 °C.

### Step 3: Preparation of PINP calibrators

The PINP antigen A1-PINP was diluted to reach concentrations at, such as 0 ng/mL, 5 ng/mL, 10 ng/mL, 20 ng/mL, 50 ng/mL, 100 ng/mL, 300 ng/mL, 500 ng/mL, 1000 ng/mL, 1600 ng/mL, and 2200 ng/mL, etc. respectively, thereby obtaining PINP calibrators.

### Step 4: Measurement procedure of tPINP kit

① The tPINP kit (including R1 reagent and R2 reagent) was loaded into the Mindray CL series fully automatic chemiluminescence immunoassay analyzer;
② The system required a sample volume of 5 µL for each measurement, and the total measurement time for each report was 17.5 minutes;

The specific sample addition process of the detection system was as follows: the fully automatic chemiluminescence immunoassay analyzer was used, 5 µL of sample, 50 µL of R1 reagent and 50 µL of R2 reagent were added to reaction tubes, respectively. After incubation, the PINP in the sample bound to the anti-PINP antibody coated on the magnetic beads, and at the same time, the anti-PINP antibody-alkaline phosphatase conjugate bound to another site of the PINP in the sample to form a sandwich complex. After the reaction was complete, the magnetic beads were attracted by a magnetic field, and the unbound substances were washed away.

Then a reaction substrate or excitation solution was added into the reaction tube to generate a light signal, and the number of photons generated was measured. The number of photons generated was proportional to the concentration of PINP in the sample.

③ The corresponding PINP calibrators were used to perform the calibration assays. Through the calibration data, the system software used a weighted four-parameter logarithmic curve (4PLC) mathematical method to fit the luminescence signal and concentration. The final results were given in the concentration form of ng/mL.

### Example 8: Preparation of tPINP kit and calibrator, and tPINP detection method

### Step 1: Preparation of R1 reagent

The specific PINP antibody (hPINP05) was mixed with superparamagnetic particles in TBS buffer, allowed to react sufficiently, then placed on a magnetic separator until the supernatant was no longer turbid, then the supernatant was discarded. The superparamagnetic particles coated with PINP antibody were kept and washed three times with TBS buffer. Finally, the superparamagnetic particles coated with PINP antibody were dissolved with R1 diluent, and the final content of superparamagnetic particles was 0.07% and the content of PINP antibody was 0.002 %, wherein R1 diluent was composed of: 50 mM Tris, 0.9% NaCl, 0.048% Proclin300, 0.1% Tween-20, 1% BSA, pH 8.0, and the R1 reagent was finally obtained and stored at 2 to 8 °C.

### Step 2: Preparation of R2 reagent

Another specific PINP antibody (hPINP02) was coupled to alkaline phosphatase, wherein the molar ratio of the obtained conjugate to antibody was 15:1, and then the conjugate was dissolved with R2 diluent, and the final concentration of the conjugate was 2 µg/mL, wherein R2 diluent was composed of: 50 mM Tris, 0.9% NaCl, 0.048% Proclin300, 0.1% Tween-20, 1% BSA, pH 8.0, and finally the R2 reagent was obtained and stored at 2 to 8 °C.

### Step 3: Preparation of PINP calibrators

The PINP antigen A1-PINP was diluted to reach concentrations at, such as 0 ng/mL, 5 ng/mL, 10 ng/mL, 20 ng/mL, 50 ng/mL, 100 ng/mL, 300 ng/mL, 500 ng/mL, 1000 ng/mL, 1600 ng/mL, and 2200 ng/mL, etc. respectively, thereby obtaining PINP calibrators.

### Step 4: Measurement procedure of tPINP Kit

① The tPINP kit (including R1 reagent and R2 reagent) was loaded into the Mindray CL series fully automatic chemiluminescence immunoassay analyzer;
② The system required a sample volume of 5 µL for each measurement, and the total measurement time for each report was 17.5 minutes;

The specific sample addition process of the detection system was as follows: the fully automatic chemiluminescence immunoassay analyzer was used, 5 µL of sample, 50 µL of R1 reagent and 50 µL of R2 reagent were added to reaction tubes, respectively. After incubation, the PINP in the sample bound to the anti-PINP antibody coated on the magnetic beads, and at the same time, the anti-PINP antibody-alkaline phosphatase conjugate bound to another site of the PINP in the sample to form a sandwich complex. After the reaction was complete, the magnetic beads were attracted by a magnetic field, and the unbound substances were washed away.

Then a reaction substrate or excitation solution was added into the reaction tube to generate a light signal, and the number of photons generated was measured. The number of photons generated was proportional to the concentration of PINP in the sample.

③ The corresponding PINP calibrators were used to perform the calibration assays. Through the calibration data, the system software used a weighted four-parameter logarithmic curve (4PLC) mathematical method to fit the luminescence signal and concentration. The final results were given in the concentration form of ng/mL.

### Example 9: Preparation of tPINP kit and calibrator, and detection of tPINP

### Step 1: Preparation of R1 reagent

The specific PINP antibody (PINP01) was mixed with superparamagnetic particles in TBS buffer, allowed to react sufficiently, then placed on a magnetic separator until the supernatant was no longer turbid, then the supernatant was discarded. The superparamagnetic particles coated with PINP antibody were kept and washed three times with TBS buffer. Finally, the superparamagnetic particles coated with PINP antibody were dissolved with R1 diluent, and the final content of superparamagnetic particles was 0.07% and the content of PINP antibody was 0.002 %, wherein R1 diluent was composed of: 50 mM Tris, 0.9% NaCl, 0.048% Proclin300, 0.1% Tween-20, 1% BSA, pH 8.0, and the R1 reagent was finally obtained and stored at 2 to 8 °C.

### Step 2: Preparation of R2 reagent

Another specific PINP antibody (hPINP06) was coupled to alkaline phosphatase, wherein the molar ratio of the obtained conjugate to antibody was 15:1, and then the conjugate was dissolved with R2 diluent, and the final concentration of the conjugate was 2 µg/mL, wherein R2 diluent was composed of: 50 mM Tris, 0.9% NaCl, 0.048% Proclin300, 0.1% Tween-20, 1% BSA, pH 8.0, and finally the R2 reagent was obtained and stored at 2 to 8 °C.

### Step 3: Preparation of PINP calibrators

The PINP antigen A1-PINP was diluted to reach concentrations at, such as 0 ng/mL, 5 ng/mL, 10 ng/mL, 20 ng/mL, 50 ng/mL, 100 ng/mL, 300 ng/mL, 500 ng/mL, 1000 ng/mL, 1600 ng/mL, and 2200 ng/mL, etc. respectively, thereby obtaining PINP calibrators.

### Step 4: Measurement procedure of tPINP Kit

① The tPINP kit (including R1 reagent and R2 reagent) was loaded into the Mindray CL series fully automatic chemiluminescence immunoassay analyzer;
② The system required a sample volume of 5 µL for each measurement, and the total measurement time for each report was 17.5 minutes;

The specific sample addition process of the detection system was as follows: the fully automatic chemiluminescence immunoassay analyzer was used, 5 µL of sample, 50 µL of R1 reagent and 50 µL of R2 reagent were added to reaction tubes, respectively. After incubation, the PINP in the sample bound to the anti-PINP antibody coated on the magnetic beads, and at the same time, the anti-PINP antibody-alkaline phosphatase conjugate bound to another site of the PINP in the sample to form a sandwich complex. After the reaction was complete, the magnetic beads were attracted by a magnetic field, and the unbound substances were washed away.

Then a reaction substrate or excitation solution was added into the reaction tube to generate a light signal, and the number of photons generated was measured. The number of photons generated was proportional to the concentration of PINP in the sample.

③ The corresponding PINP calibrators were used to perform the calibration assays. Through the calibration data, the system software used a weighted four-parameter logarithmic curve (4PLC) mathematical method to fit the luminescence signal and concentration. The final results were given in the concentration form of ng/mL.

### Example 10: Preparation of tPINP kit and calibrator, and detection of tPINP

### Step 1: Preparation of R1 reagent

The specific PINP antibody (PINP01) was mixed with superparamagnetic particles in TBS buffer, allowed to react sufficiently, then placed on a magnetic separator until the supernatant was no longer turbid, then the supernatant was discarded. The superparamagnetic particles coated with PINP antibody were kept and washed three times with TBS buffer. Finally, the superparamagnetic particles coated with PINP antibody were dissolved with R1 diluent, and the final content of superparamagnetic particles was 0.07% and the content of PINP antibody was 0.002 %, wherein R1 diluent was composed of: 50 mM Tris, 0.9% NaCl, 0.048% Proclin300, 0.1% Tween-20, 1% BSA, pH 8.0, and the R1 reagent was finally obtained and stored at 2 to 8 °C.

### Step 2: Preparation of R2 reagent

Another specific PINP antibody (hPINP02) was coupled to alkaline phosphatase, wherein the molar ratio of the obtained conjugate to antibody was 15:1, and then the conjugate was dissolved with R2 diluent, and the final concentration of the conjugate was 2 µg/mL, wherein R2 diluent was composed of: 50 mM Tris, 0.9% NaCl, 0.048% Proclin300, 0.1% Tween-20, 1% BSA, pH 8.0, and finally the R2 reagent was obtained and stored at 2 to 8 °C.

### Step 3: Preparation of PINP calibrators

The PINP antigen A1-PINP was diluted to reach concentrations at, such as 0 ng/mL, 5 ng/mL, 10 ng/mL, 20 ng/mL, 50 ng/mL, 100 ng/mL, 300 ng/mL, 500 ng/mL, 1000 ng/mL, 1600 ng/mL, and 2200 ng/mL, etc. respectively, thereby obtaining PINP calibrators.

### Step 4: Measurement procedure of tPINP Kit

① The tPINP kit (including R1 reagent and R2 reagent) was loaded into the Mindray CL series fully automatic chemiluminescence immunoassay analyzer;
② The system required a sample volume of 5 µL for each measurement, and the total measurement time for each report was 17.5 minutes;

The specific sample addition process of the detection system was as follows: the fully automatic chemiluminescence immunoassay analyzer was used, 5 µL of sample, 50 µL of R1 reagent and 50 µL of R2 reagent were added to reaction tubes, respectively. After incubation, the PINP in the sample bound to the anti-PINP antibody coated on the magnetic beads, and at the same time, the anti-PINP antibody-alkaline phosphatase conjugate bound to another site of the PINP in the sample to form a sandwich complex. After the reaction was complete, the magnetic beads were attracted by a magnetic field, and the unbound substances were washed away.

Then a reaction substrate or excitation solution was added into the reaction tube to generate a light signal, and the number of photons generated was measured. The number of photons generated was proportional to the concentration of PINP in the sample.

③ The corresponding PINP calibrators were used to perform the calibration assays. Through the calibration data, the system software used a weighted four-parameter logarithmic curve (4PLC) mathematical method to fit the luminescence signal and concentration. The final results were given in the concentration form of ng/mL.

### Example 11: Preparation of tPINP kit and calibrator, and detection of tPINP

### Step 1: Preparation of R1 reagent

The specific PINP antibody (hPINP03) was mixed with superparamagnetic particles in TBS buffer, allowed to react sufficiently, then placed on a magnetic separator until the supernatant was no longer turbid, then the supernatant was discarded. The superparamagnetic particles coated with PINP antibody were kept and washed three times with TBS buffer. Finally, the superparamagnetic particles coated with PINP antibody were dissolved with R1 diluent, and the final content of superparamagnetic particles was 0.07% and the content of PINP antibody was 0.002 %, wherein R1 diluent was composed of: 50 mM Tris, 0.9% NaCl, 0.048% Proclin300, 0.1% Tween-20, 1% BSA, pH 8.0, and the R1 reagent was finally obtained and stored at 2 to 8 °C.

### Step 2: Preparation of R2 reagent

Another specific PINP antibody (hPINP02) was coupled to alkaline phosphatase, wherein the molar ratio of the obtained conjugate to antibody was 15:1, and then the conjugate was dissolved with R2 diluent, and the final concentration of the conjugate was 2 µg/mL, wherein R2 diluent was composed of: 50 mM Tris, 0.9% NaCl, 0.048% Proclin300, 0.1% Tween-20, 1% BSA, pH 8.0, and finally the R2 reagent was obtained and stored at 2 to 8 °C.

### Step 3: Preparation of PINP calibrators

The PINP antigen A1-PINP was diluted to reach concentrations at, such as 0 ng/mL, 5 ng/mL, 10 ng/mL, 20 ng/mL, 50 ng/mL, 100 ng/mL, 300 ng/mL, 500 ng/mL, 1000 ng/mL, 1600 ng/mL, and 2200 ng/mL, etc. respectively, thereby obtaining PINP calibrators.

### Step 4: Measurement procedure of tPINP Kit

① The tPINP kit (including R1 reagent and R2 reagent) was loaded into the Mindray CL series fully automatic chemiluminescence immunoassay analyzer;
② The system required a sample volume of 5 µL for each measurement, and the total measurement time for each report was 17.5 minutes;

The specific sample addition process of the detection system was as follows: the fully automatic chemiluminescence immunoassay analyzer was used, 5 µL of sample, 50 µL of R1 reagent and 50 µL of R2 reagent were added to reaction tubes, respectively. After incubation, the PINP in the sample bound to the anti-PINP antibody coated on the magnetic beads, and at the same time, the anti-PINP antibody-alkaline phosphatase conjugate bound to another site of the PINP in the sample to form a sandwich complex. After the reaction was complete, the magnetic beads were attracted by a magnetic field, and the unbound substances were washed away.

Then a reaction substrate or excitation solution was added into the reaction tube to generate a light signal, and the number of photons generated was measured. The number of photons generated was proportional to the concentration of PINP in the sample.

③ The corresponding PINP calibrators were used to perform the calibration assays. Through the calibration data, the system software used a weighted four-parameter logarithmic curve (4PLC) mathematical method to fit the luminescence signal and concentration. The final results were given in the concentration form of ng/mL.

### Detection items and results:

### 1. Minimum detection limit

A sample with concentration of zero was used as a tested sample to carry out the detection, and the measurement was repeated 20 times to obtain the RLU value (relative luminescence value) of the 20 measurement results, mean (M) and standard deviation (SD) were calculated. For a reagent, a linear equation (E2) was obtained by two-point regression fitting according to the results of concentrations between zero-concentration calibrator and adjacent calibrator of the master calibration curve and the RLU value, and the RLU value corresponding to M+2SD was brought into the above equation E2 to obtain the corresponding concentration, which was the minimum detection limit. The detection capability of the current kit could reach 0.03 ng/mL.

| | Example 6 | Example 7 | Example 8 | Example 9 | Example 10 | Example 11 |
|---|---|---|---|---|---|---|
| Sample | RLU | RLU | RLU | RLU | RLU | RLU |
| | 3974 | 3944 | 4394 | 4413 | 4155 | 4110 |
| | 4018 | 3897 | 4472 | 4462 | 4194 | 4153 |
| | 3974 | 3990 | 4403 | 4480 | 4350 | 4127 |
| | 4072 | 3993 | 4415 | 4494 | 4218 | 4122 |
| | 4039 | 3983 | 4466 | 4482 | 4176 | 4275 |
| | 4112 | 3935 | 4573 | 4474 | 4273 | 4175 |
| | 4032 | 4062 | 4480 | 4517 | 4300 | 4280 |
| | 4053 | 4160 | 4473 | 4517 | 4245 | 4091 |
| | 3972 | 4006 | 4459 | 4499 | 4164 | 4253 |
| | 4072 | 4043 | 4441 | 4506 | 4217 | 4113 |
| | 4062 | 4025 | 4418 | 4454 | 4214 | 4010 |
| | 3990 | 3979 | 4524 | 4492 | 4301 | 4025 |
| | 4014 | 3999 | 4451 | 4561 | 4187 | 4050 |
| | 4032 | 4093 | 4491 | 4529 | 4223 | 4081 |
| | 4078 | 4002 | 4473 | 4796 | 4239 | 4049 |
| | 4078 | 4046 | 4443 | 4518 | 4205 | 3999 |
| | 4032 | 4025 | 4484 | 4491 | 4249 | 4012 |
| | 4062 | 3997 | 4512 | 4450 | 4195 | 4116 |
| | 4114 | 4064 | 4468 | 4482 | 4176 | 4071 |
| | 4031 | 3946 | 4412 | 4475 | 4352 | 4255 |
| Mean | 4041 | 4009 | 4463 | 4505 | 4232 | 4118 |
| Standard deviation | 42 | 60 | 44 | 76 | 58 | 89 |
| M+2SD | 4125 | 4129 | 4550 | 4656 | 4347 | 4297 |
| M+2SD concentration (ng/mL) | 0.03 | 0.04 | 0.04 | 0.05 | 0.04 | 0.04 |

### 2. Detection range and linearity

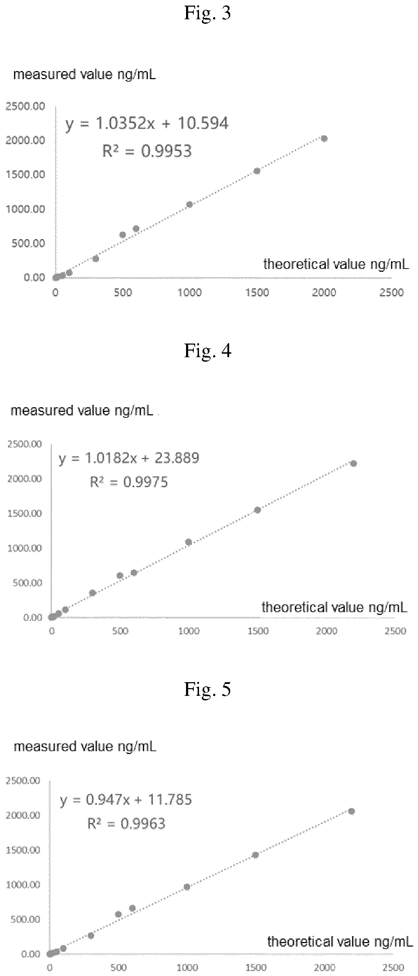

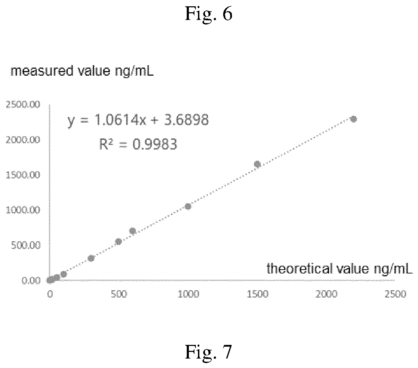

A quantitative detection method by establishing a linear range was used, in which 11 concentration levels should be selected within the expected detection range. High-value samples close to the upper limit of the linear range were diluted in certain ratios to several concentrations, and low-value samples had concentrations close to the lower limit of the linear range. The measurement results and the theoretical results were subjected to straight line fitting, and the correlation coefficient R² within the linear range was calculated. The results were shown in Fig. 2 to Fig. 7.

The results showed that the current kits had a detection range of more than 2000 ng/mL, and the linear R² was greater than 0.99 between 0.03 ng/mL and 2000 ng/mL.

### 3. Specificity

The specificity evaluation was carried out mainly to evaluate the interference of serum endogenous interfering substances: hemolysis (hemoglobin), high fat, high bilirubin, total protein serum endogenous interfering substances, as well as the interference of common analogues of P1NP.

Interfering drugs were added to serum to prepare interfering substance-containing serum samples, respectively. At the same time, the solvent of each interfering substance and basic serum were used to prepare a control serum sample according to the method for preparing the corresponding interfering substance-containing sample. The interfering substance-containing samples and control serum samples were detected, respectively. The mean value of detecting the interfering substance-containing sample was recorded as M, the mean value of detecting the control serum sample was recorded as T, and the relative deviation B of the measured concentration was calculated according to the formula: B=(M-T)/T×100%. The results were shown in the table below.

| Specificity | Example 6 | Example 7 | Example 8 | Example 9 | Example 10 | Example 11 |
|---|---|---|---|---|---|---|
| Parathyroid hormone | 1.20% | 3.57% | -2.87% | 2.75% | 4.34% | -4.66% |
| Osteocalcin | 3.99% | 0.19% | -4.58% | 3.49% | 0.17% | -4.43% |
| Vitamin D | 3.69% | 1.60% | -3.09% | -1.88% | 3.87% | -3.52% |
| Calcitonin | 0.40% | 4.24% | -1.71% | -0.23% | 4.90% | -3.46% |
| Triglycerides | 1.17% | 2.66% | 3.48% | 2.39% | -0.80% | -1.84% |
| Bilirubin | 0.98% | 2.01% | 0.28% | -0.12% | -0.17% | 2.04% |
| Hemoglobin | 0.20% | 0.73% | -0.74% | -0.94% | 0.23% | -1.54% |
| Total protein | -3.82% | -4.12% | -4.58% | -4.50% | -0.81% | -0.99% |
| Biotin | 1.62% | 1.07% | 1.39% | -0.10% | 1.00% | 1.82% |

The results showed that the specificity deviations of the current kits were within 5%, indicating that the prepared kits had strong anti-interference ability.

Information of the sequences involved in the present application is described in the table below:

| SEQ ID NO: | Description | Sequence |
|---|---|---|
| 1 | PINP01 Kabat CDR-H1 amino acid sequence | SYWMN |
| 2 | PINP01 Kabat CDR-H2 amino acid sequence | MIHPSDSETRLNQKFKD |
| 3 | PINP01 Kabat CDR-H3 amino acid sequence | THIYYVFDY |
| 4 | PINP01 Kabat CDR-L1 amino acid sequence | KSSQSLLNSRTRKNYLA |
| 5 | PINP01 Kabat CDR-L2 amino acid sequence | WASTRES |
| 6 | PINP01 Kabat CDR-L3 amino acid sequence | KQSYNLYT |
| 7 | hPINP02 Kabat CDR-H1 amino acid sequence | NAAMY |
| 8 | hPINP02 Kabat CDR-H2 amino acid sequence | RIRTKPNNYATYYADSVKG |
| 9 | hPINP02 Kabat CDR-H3 amino acid sequence | TTTDMGPMGY |
| 10 | hPINP02 Kabat CDR-L1 amino acid sequence | RASQDIGNYLR |
| 11 | hPINP02 Kabat CDR-L2 amino acid sequence | GETNLAN |
| 12 | hPINP02 Kabat CDR-L3amino acid sequence | LQHYDYPLT |
| 13 | hPINP03 Kabat CDR-H1 amino acid sequence | NNGVS |
| 14 | hPINP03 Kabat CDR-H2 amino acid sequence | AMSSGGNTYYNSGLKS |
| 15 | hPINP03 Kabat CDR-H3 amino acid sequence | HDAGLIY |
| 16 | hPINP03 Kabat CDR-L1 amino acid sequence | KASQNVGTNVD |
| 17 | hPINP03 Kabat CDR-L2 amino acid sequence | GASNRYT |
| 18 | hPINP03 Kabat CDR-L3 amino acid sequence | LQYNDTPWT |
| 19 | hPINP04 Kabat CDR-H1 amino acid sequence | DYYMA |
| 20 | hPINP04 Kabat CDR-H2 amino acid sequence | TISYDGSGTHYRDSVKG |
| 21 | hPINP04 Kabat CDR-H3 amino acid sequence | RRGYGYYYVMDA |
| 22 | hPINP04 Kabat CDR-L1 amino acid sequence | KASQNVGSNVD |
| 23 | hPINP04 Kabat CDR-L2 amino acid sequence | KASNLYT |
| 24 | hPINP04 Kabat CDR-L3 amino acid sequence | MQSGSYPPT |
| 25 | hPINP05 Kabat CDR-H1 amino acid sequence | NAAMY |
| 26 | hPINP05 Kabat CDR-H2 amino acid sequence | RIRTKVNNYATYYADSVRG |
| 27 | hPINP05 Kabat CDR-H3 amino acid sequence | AGYYDGTYYYEGDYFDY |
| 28 | hPINP05 Kabat CDR-L1 amino acid sequence | RASQSVSITGYSLMH |
| 29 | hPINP05 Kabat CDR-L2 amino acid sequence | RTSDLAS |
| 30 | hPINP05 Kabat CDR-L3 amino acid sequence | QQSRESPPT |
| 31 | hPINP06 Kabat CDR-H1 amino acid sequence | NAAMY |
| 32 | hPINP06 Kabat CDR-H2 amino acid sequence | RIRTKPNDYATYYTDSVKG |
| 33 | hPINP06 Kabat CDR-H3 amino acid sequence | ETRNLDWFAY |
| 34 | hPINP06 Kabat CDR-L1 amino acid sequence | RSSKSPRHSNGITYVY |
| 35 | hPINP06 Kabat CDR-L2 amino acid sequence | RMSNLAS |
| 36 | hPINP06 Kabat CDR-L3 amino acid sequence | GQVLENLPT |
| 37 | hPINP07 Kabat CDR-H1 amino acid sequence | GSSVGVG |
| 38 | hPINP07 Kabat CDR-H2 amino acid sequence | TIGWEDVKHYNPSLKS |
| 39 | hPINP07 Kabat CDR-H3 amino acid sequence | SWERFDY |
| 40 | hPINP07 Kabat CDR-L1 amino acid sequence | SGDELPKRYAY |
| 41 | hPINP07 Kabat CDR-L2 amino acid sequence | KDSERPS |
| 42 | hPINP07 Kabat CDR-L3 amino acid sequence | HSTYSDDKLPV |
| 43 | A1-PINP | MFSFVDLRLLLLLAATALLTHGQEEGQVEGQDED IPPITCVQNGLRYHDRDVWKPEPCRICVCDNGKVL CDDVICDETKNCPGAEVPEGECCPVCPDGSESPTD QETTGVEGPKGDTGPRGPRGPAGPPGRDGIPGQPG LPGPPGPPGPPGPPGLGGNFAPGGGSGGGSGGGSH HHHHH |
| 44 | PINP01 Kabat CDR-H1 nucleotide sequence | AGCTACTGGATGAAC |
| 45 | PINP01 Kabat CDR-H2 nucleotide sequence | ATGATTCATCCTTCCGATAGTGAAACTAGGTTA AATCAGAAGTTCAAGGAC |
| 46 | PINP01 Kabat CDR-H3 nucleotide sequence | ACCCACATCTACTATGTCTTTGACTAC |
| 47 | PINP01 Kabat CDR-L1 nucleotide sequence | AAATCCAGTCAGAGTCTGCTCAACAGTAGAACC CGAAAGAACTACTTGGCT |
| 48 | PINP01 Kabat CDR-L2 nucleotide sequence | TGGGCATCCACTAGGGAATCT |
| 49 | PINP01 Kabat CDR-L3 nucleotide sequence | AAGCAATCTTATAATCTGTACACG |
| 50 | hPINP02 Kabat CDR-H1 nucleotide sequence | AATGCTGCCATGTAC |
| 51 | hPINP02 Kabat CDR-H2 nucleotide sequence | CGCATAAGAACTAAACCTAATAATTATGCAACA TATTATGCTGATTCAGTGAAAGGC |
| 52 | hPINP02 Kabat CDR-H3 nucleotide sequence | ACGACTACGGATATGGGTCCTATGGGGTAC |
| 53 | hPINP02 Kabat CDR-L1 nucleotide sequence | CGGGCAAGTCAAGACATTGGAAATTATTTAAGA |
| 54 | hPINP02 Kabat CDR-L2 nucleotide sequence | GGTGAAACCAACTTGGCAAAT |
| 55 | hPINP02 Kabat CDR-L3 nucleotide sequence | CTGCAGCATTATGATTATCCTCTCACG |
| 56 | hPINP03 Kabat CDR-H1 nucleotide sequence | AACAATGGTGTAAGC |
| 57 | hPINP03 Kabat CDR-H2 nucleotide sequence | GCAATGTCAAGTGGTGGAAACACATATTATAAT TCAGGTCTCAAATCC |
| 58 | hPINP03 Kabat CDR-H3 nucleotide sequence | CACGACGCGGGGCTAATTTAC |
| 59 | hPINP03 Kabat CDR-L1 nucleotide sequence | AAGGCCAGTCAGAATGTGGGTACTAATGTAGAC |
| 60 | hPINP03 Kabat CDR-L2 nucleotide sequence | GGGGCATCCAACCGGTACACT |
| 61 | hPINP03 Kabat CDR-L3 nucleotide sequence | CTACAGTATAACGACACTCCGTGGACG |
| 62 | hPINP04 Kabat CDR-H1 nucleotide sequence | GACTATTACATGGCC |
| 63 | hPINP04 Kabat CDR-H2 nucleotide sequence | ACCATTAGTTATGATGGTAGTGGCACTCACTAT CGAGACTCCGTGAAGGGC |
| 64 | hPINP04 Kabat CDR-H3 nucleotide sequence | CGAAGGGGTTACGGCTATTACTATGTTATGGAT GCC |
| 65 | hPINP04 Kabat CDR-L1 nucleotide sequence | AAGGCCAGTCAAAATGTGGGTTCTAATGTAGAC |
| 66 | hPINP04 Kabat CDR-L2 nucleotide sequence | AAAGCATCCAACCTGTACACG |
| 67 | hPINP04 Kabat CDR-L3 nucleotide sequence | ATGCAGTCTGGCTCCTATCCTCCAACG |
| 68 | hPINP05 Kabat CDR-H1 nucleotide sequence | AATGCTGCCATGTAC |
| 69 | hPINP05 Kabat CDR-H2 nucleotide sequence | CGCATAAGAACTAAAGTTAATAATTATGCAACA TATTATGCTGATTCAGTGAGAGGC |
| 70 | hPINP05 Kabat CDR-H3 nucleotide sequence | GCGGGCTATTATGATGGTACTTATTATTACGAG GGCGACTACTTTGATTAC |
| 71 | hPINP05 Kabat CDR-L1 nucleotide sequence | AGGGCCAGCCAAAGTGTCAGTATAACTGGATAT AGTCTTATGCAC |
| 72 | hPINP05 Kabat CDR-L2 nucleotide sequence | CGTACATCCGACCTAGCATCT |
| 73 | hPINP05 Kabat CDR-L3 nucleotide sequence | CAGCAGAGTAGGGAGTCTCCTCCGACG |
| 74 | hPINP06 Kabat CDR-H1 nucleotide sequence | AATGCTGCCATGTAC |
| 75 | hPINP06 Kabat CDR-H2 nucleotide sequence | CGCATAAGAACTAAACCTAATGATTATGCAACA TATTACACTGATTCAGTGAAAGGC |
| 76 | hPINP06 Kabat CDR-H3 nucleotide sequence | GAGACTCGCAACTTGGATTGGTTTGCTTAC |
| 77 | hPINP06 Kabat CDR-L1 nucleotide sequence | AGATCTAGTAAGAGTCCGCGACATAGTAATGGC ATCACTTACGTGTAT |
| 78 | hPINP06 Kabat CDR-L2 nucleotide sequence | CGGATGTCCAACCTTGCCTCA |
| 79 | hPINP06 Kabat CDR-L3 nucleotide sequence | GGACAGGTTCTAGAAAATCTTCCGACG |
| 80 | hPINP07 Kabat CDR-H1 nucleotide sequence | GGTTCCAGTGTGGGCGTGGGC |
| 81 | hPINP07 Kabat CDR-H2 nucleotide sequence | ACCATTGGGTGGGAGGATGTTAAGCACTACAAT CCATCTCTGAAGAGC |
| 82 | hPINP07 Kabat CDR-H3 nucleotide sequence | TCCTGGGAGAGGTTTGATTAC |
| 83 | hPINP07 Kabat CDR-L1 nucleotide sequence | TCTGGAGATGAGTTACCAAAAAGATATGCTTAT |
| 84 | hPINP07 Kabat CDR-L2 nucleotide sequence | AAAGATAGTGAGCGGCCCTCA |
| 85 | hPINP07 Kabat CDR-L3 nucleotide sequence | CACTCGACATATAGTGATGATAAACTCCCTGTT |

Although the specific embodiments of the present invention have been described in detail, those skilled in the art will understand that various modifications and substitutions can be made to those details based on all teachings that have been disclosed, and these changes are within the scope of the present invention. The full scope of the present invention is given by the appended claims and any equivalents thereof.

## Claims

1. An antibody or antigen-binding fragment thereof that specifically binds to PINP (Procollagen I N-Terminal Propeptide) or different forms of existence thereof, comprising:
a heavy chain variable region comprising following three CDRs: CDR-H1 having an amino acid sequence selected from SEQ ID NO: 1, 7, 13, 19, 25, 31 and 37 or a variant thereof, CDR-H2 having an amino acid sequence selected from SEQ ID NO: 2, 8, 14, 20, 26, 32 and 38 or a variant thereof, and/or CDR-H3 having an amino acid sequence selected from SEQ ID NO: 3, 9, 15, 21, 27, 33 and 39 or a variant thereof; and/or,
a light chain variable region comprising following three CDRs: CDR-L1 having an amino acid sequence selected from SEQ ID NO: 4, 10, 16, 22, 28, 34 and 40 or a variant thereof, CDR-L2 having an amino acid sequence selected from SEQ ID NO: 5, 11, 17, 23, 29, 35 and 41 or a variant thereof, and/or CDR-L3 having an amino acid sequence selected from SEQ ID NO: 6, 12, 18, 24, 30, 36 and 42 or a variant thereof;
wherein, each variant contains an amino acid mutation, the mutation is substitution, deletion or addition of one or more amino acids (for example, the substitution, deletion or addition of 1, 2, 3, 4 or 5 amino acids) as compared to an amino acid sequence from which said variant is derived; preferably, the substitution is a conservative substitution; preferably, said variant has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which said variant is derived;
preferably, the three CDRs of the heavy chain variable region, and/or the three CDRs of the light chain variable region are/is defined by Kabat, Chothia, or IMGT numbering system;
preferably, the antibody or antigen-binding fragment thereof specifically binds to an epitope contained in a sequence of amino acid residues 23 to 161 of COL1A1 (human Collagen I α1).

2. The antibody or antigen-binding fragment thereof according to claim 1, wherein:
(1a) the heavy chain variable region comprises following three CDRs: CDR-H1 having an amino acid sequence of SEQ ID NO: 1 or a variant thereof, CDR-H2 having an amino acid of SEQ ID NO: 2 or a variant thereof, and CDR-H3 having an amino acid sequence of SEQ ID NO: 3 or a variant thereof; and the light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;
preferably, the antibody or antigen-binding fragment thereof: (a1) is produced by a hybridoma cell line deposited with the All-Russian National Collection of Industrial Microorganisms (VKPM) having Accession Number H-214; (a2) comprises same amino acid sequences as those of CDR-H1, CDR-H2 and CDR-H3 of a heavy chain variable region and/or those of CDR-L1, CDR-L2 and CDR-L3 of a light chain variable region of an antibody produced by the hybridoma cell line deposited with VKPM having Accession Number H-214; (a3) comprises same amino acid sequence as that of a heavy chain variable region and/or that of a light chain variable region of an antibody produced by the hybridoma cell line deposited with VKPM having Accession Number H-214; or (a4) comprise same amino acid sequence as that of a heavy chain and/or that of a light chain of an antibody produced by the hybridoma cell line deposited with VKPM having Accession Number H-214;
(1b) the heavy chain variable region comprises following three CDRs: CDR-H1 having an amino acid sequence of SEQ ID NO: 7 or a variant thereof, CDR-H2 having an amino acid of SEQ ID NO: 8 or a variant thereof, and CDR-H3 having an amino acid sequence of SEQ ID NO: 9 or a variant thereof; and the light chain variable region comprises following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;
preferably, the antibody or antigen-binding fragment thereof: (b1) comprises same amino acid sequence as an amino acid sequence encoded by an inserted portion of plasmid deposited with VKPM having Accession Number B-14744 and/or an amino acid sequence encoded by an inserted portion of plasmid deposited with VKPM having Accession Number B-14745; (b2) of which the heavy chain comprises same amino acid sequence as an amino acid sequence encoded by an inserted of plasmid deposited with VKPM having Accession Number B-14744 and/or the light chain comprises same amino acid sequence as an amino acid sequence encoded by an inserted portion of plasmid deposited with VKPM having Accession Number B-14745; or (b3) comprises same amino acid sequences as those of CDR-H1, CDR-H2 and CDR-H3 set forth in an amino acid sequence encoded by an inserted portion of plasmid deposited with VKPM having Accession Number B-14744 and/or those of CDR-L1, CDR-L2 and CDR-L3 set forth in an amino acid sequence encoded by an inserted portion of plasmid deposited with VKPM having Accession Number B-14745;
(1c) the heavy chain variable region comprises the following three CDRs: CDR-H1 having an amino acid sequence of SEQ ID NO: 13 or a variant thereof, CDR-H2 having an amino acid of SEQ ID NO: 14 or a variant thereof, and CDR-H3 having an amino acid sequence of SEQ ID NO: 15 or a variant thereof; and the light chain variable region comprises following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;
preferably, the antibody or antigen-binding fragment thereof: (c1) comprises same amino acid sequence as an amino acid sequence encoded by an inserted portion of plasmid deposited with VKPM having Accession Number B-14746 and/or an amino acid sequence encoded by an inserted portion of plasmid deposited with VKPM having Accession Number B-14747; (c2) of which the heavy chain comprises same amino acid sequence as an amino acid sequence encoded by an inserted of plasmid deposited with VKPM having Accession Number B-14746 and/or the light chain comprises same amino acid sequence as an amino acid sequence encoded by an inserted portion of plasmid deposited with VKPM having Accession Number B-14747; or (c3) comprises same amino acid sequences as those of CDR-H1, CDR-H2 and CDR-H3 set forth in an amino acid sequence encoded by an inserted portion of plasmid deposited with VKPM having Accession Number B-14746 and/or those of CDR-L1, CDR-L2 and CDR-L3 set forth in an amino acid sequence encoded by an inserted portion of plasmid deposited with VKPM having Accession Number B-14747;
(1d) the heavy chain variable region comprises following three CDRs: CDR-H1 having an amino acid sequence of SEQ ID NO: 19 or a variant thereof, CDR-H2 having an amino acid of SEQ ID NO: 20 or a variant thereof, and CDR-H3 having an amino acid sequence of SEQ ID NO: 21 or a variant thereof; and the light chain variable region comprises following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;
preferably, the antibody or antigen-binding fragment thereof: (d1) comprises same amino acid sequence as an amino acid sequence encoded by an inserted portion of plasmid deposited with VKPM having Accession Number B-14748 and/or an amino acid sequence encoded by an inserted portion of plasmid deposited with VKPM having Accession Number B-14749; (d2) of which the heavy chain comprises same amino acid sequence as an amino acid sequence encoded by an inserted of plasmid deposited with VKPM having Accession Number B-14748 and/or the light chain comprises same amino acid sequence as an amino acid sequence encoded by an inserted portion of plasmid deposited with VKPM having Accession Number B-14749; or (d3) comprises same amino acid sequences as those of CDR-H1, CDR-H2 and CDR-H3 set forth in an amino acid sequence encoded by an inserted portion of plasmid deposited with VKPM having Accession Number B-14748 and/or those of CDR-L1, CDR-L2 and CDR-L3 set forth in an amino acid sequence encoded by an inserted portion of plasmid deposited with VKPM having Accession Number B-14749;
(1e) the heavy chain variable region comprises three CDRs: CDR-H1 having an amino acid sequence of SEQ ID NO: 25 or a variant thereof, CDR-H2 having an amino acid of SEQ ID NO: 26 or a variant thereof, and CDR-H3 having an amino acid sequence of SEQ ID NO: 27 or a variant thereof; and the light chain variable region comprises three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof;
preferably, the antibody or antigen-binding fragment thereof: (e1) comprises same amino acid sequence as an amino acid sequence encoded by an inserted portion of plasmid deposited with VKPM having Accession Number B-14750 and/or an amino acid sequence encoded by an inserted portion of plasmid deposited with VKPM having Accession Number B-14751; (e2) of which the heavy chain comprises same amino acid sequence as an amino acid sequence encoded by an inserted of plasmid deposited with VKPM having Accession Number B-14750 and/or the light chain comprises same amino acid sequence as an amino acid sequence encoded by an inserted portion of plasmid deposited with VKPM having Accession Number B-14751; or (e3) comprises same amino acid sequences as those of CDR-H1, CDR-H2 and CDR-H3 set forth in an amino acid sequence encoded by an inserted portion of plasmid deposited with VKPM having Accession Number B-14750 and/or those of CDR-L1, CDR-L2 and CDR-L3 set forth in an amino acid sequence encoded by an inserted portion of plasmid deposited with VKPM having Accession Number B-14751;
(1f) the heavy chain variable region comprises following three CDRs: CDR-H1 having an amino acid sequence of SEQ ID NO: 31 or a variant thereof, CDR-H2 having an amino acid of SEQ ID NO: 32 or a variant thereof, and CDR-H3 having an amino acid sequence of SEQ ID NO: 33 or a variant thereof; and the light chain variable region comprises following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof;
preferably, the antibody or antigen-binding fragment thereof: (f1) comprises same amino acid sequence as an amino acid sequence encoded by an inserted portion of plasmid deposited with VKPM having Accession Number B-14752; or (f2) comprises same amino acid sequences as those of CDR-H1, CDR-H2 and CDR-H3 set forth in an amino acid sequence encoded by an inserted portion of plasmid deposited with VKPM having Accession Number B-14752 and/or those of CDR-L1, CDR-L2 and CDR-L3 set forth in an amino acid sequence encoded by an inserted portion of plasmid deposited with VKPM having Accession Number B-14752;
or
(1g) the heavy chain variable region comprises following three CDRs: CDR-H1 having an amino acid sequence of SEQ ID NO: 37 or a variant thereof, CDR-H2 having an amino acid of SEQ ID NO: 38 or a variant thereof, and CDR-H3 having an amino acid sequence of SEQ ID NO: 39 or a variant thereof; and the light chain variable region comprises following three CDRs: CDR-L1 having an amino acid sequence of SEQ ID NO: 40 or a variant thereof, CDR-L2 having an amino acid sequence of SEQ ID NO: 41 or a variant thereof, and CDR-L3 having an amino acid sequence of SEQ ID NO: 42 or a variant thereof;
preferably, the antibody or antigen-binding fragment thereof: (g1) comprises same amino acid sequence as an amino acid sequence encoded by an inserted portion of plasmid deposited with VKPM having Accession Number B-14753; or (g2) comprises same amino acid sequences as those of CDR-H1, CDR-H2 and CDR-H3 set forth in an amino acid sequence encoded by an inserted portion of plasmid deposited with VKPM having Accession Number B-14753 and/or those of CDR-L1, CDR-L2 and CDR-L3 set forth in an amino acid sequence encoded by an inserted portion of plasmid deposited with VKPM having Accession Number B-14753.

3. The antibody or antigen-binding fragment thereof according to claim 1 or 2, further comprising a heavy chain constant region (CH) and a light chain constant region (CL);
preferably, the heavy chain constant region is a mouse heavy chain constant region and the light chain constant region is a mouse light chain constant region;
preferably, the heavy chain constant region is a human heavy chain constant region and the light chain constant region is a human light chain constant region;
preferably, the antibody or antigen-binding fragment thereof is IgG, IgM, IgE, IgD or IgA;
preferably, the heavy chain constant region is an IgG heavy chain constant region (for example, an IgG1, IgG2, IgG3 or IgG4 heavy chain constant region);
preferably, the light chain constant region is a κ or λ light chain constant region (for example, a human λ light chain constant region).

4. The antibody or antigen-binding fragment thereof according to any one of claims 1-3, wherein, the antigen-binding fragment thereof is selected from scFv, Fab, Fab', (Fab')₂, Fv fragment, disulfide bond-linked Fv(dsFv), diabody, bi-specific antibody and multi-specific antibody; and/or, the antibody is a murine antibody, chimeric antibody or humanized antibody.

5. The antibody or antigen-binding fragment thereof according to any one of claims 1-4, which has a detectable label;
preferably, the label is selected from the group consisting of fluorescein, chemiluminescence (for example, acridinium esters), enzyme (for example, horseradish peroxidase, alkaline phosphatase), radioisotope, biotin, colloidal gold, and magnetic particles.

6. An antibody or antigen-binding fragment thereof that specifically binds to PINP or different forms of existence thereof, wherein the antibody or antigen-binding fragment thereof is:
produced by a hybridoma cell line deposited with the All-Russian National Collection of Industrial Microorganisms (VKPM) having Accession Number H-214;
produced by plasmid deposited with VKPM having Accession Number B-14744 and plasmid deposited with VKPM having Accession Number B-14745;
produced by plasmid deposited with VKPM having Accession Number B-14746 and plasmid deposited with VKPM having Accession Number B-14747;
produced by plasmid deposited with VKPM having Accession Number B-14748 and plasmid deposited with VKPM having Accession Number B-14749;
produced by plasmid deposited with VKPM having Accession Number B-14750 and plasmid deposited with VKPM having Accession Number B-14751;
produced by plasmid deposited with VKPM having Accession Number B-14752; or
produced by plasmid deposited with VKPM having Accession Number B-14753.

7. An isolated nucleic acid molecule, which encodes the antibody or antigen-binding fragment thereof according to any one of claims 1 to 6.

8. An expression vector comprising the isolated nucleic acid molecule according to claim 7;
preferably, the vector is a plasmid, a virus, a bacteriophage, a bacterium or a viroid.

9. A host cell comprising the isolated nucleic acid molecule according to claim 6 or the expression vector according to claim 8;
preferably, the host cell is an eukaryotic cell, preferably a mammalian cell;
preferably, the host cell is a prokaryotic cell, preferably a colibacillus.

10. A PINP detection kit, having a detection sensitivity of ≤0.3ng/ml for tPINP (total Procollagen I N-Terminal Propeptide), e.g., ≤0.2ng/ml, ≤0.15ng/ml, <_0. 1ng/ml, ≤0.05ng/ml, or ≤0.03ng/ml;
optionally, the PINP detection kit has a detection range of 0.3-2000 ng/ml for tPINP, e.g., 0.05-2000ng/ml, 0.1-2000ng/ml, or 0.15-2000ng/ml.

11. A composition or PINP detection kit, comprising the antibody or antigen-binding fragment thereof according to any one of claims 1-6;
preferably, the composition or PINP detection kit comprises:
a first antibody, which is selected from the antibody or antigen-binding fragment thereof according to any one of claims 1-6, and,
a second antibody, which is selected from the antibody or antigen-binding fragment thereof according to any one of claims 1-6;
wherein, the first antibody and the second antibody respectively target different epitopes of PINP;
preferably, the composition or PINP detection kit is **characterized by** any one of items I) -VII):
I) the first antibody is the antibody of (1a) of claim 2 or the antigen-binding fragment thereof;
the second antibody is selected from the group consisting of the following antibodies or antigen-binding fragments thereof as defined in claim 2: (1b), (1c), (1d), (1e), (1f) and (1g), preferably is (1b), (1d), (1e) or (1f), more preferably is (1b), (1d) or (1f);
II) the first antibody is the antibody of (1b) of claim 2 or the antigen-binding fragment thereof;
the second antibody is selected from the group consisting of the following antibodies or antigen-binding fragments thereof as defined in claim 2: (1a), (1c), (1d), (1e), (1f) and (1g), preferably (1a), (1c), (1d), (1e) or (1g), more preferably (1g);
III) the first antibody is the antibody of (1c) of claim 2 or antigen-binding fragment thereof;
the second antibody is selected from the group consisting of the following antibodies or antigen-binding fragment thereof as defined in claim 2: (1a), (1b), (1d), (1e), (1f), and (1g), preferably (1b), (1e) or (1f);
IV) the first antibody is the antibody of (1d) of claim 2 or antigen-binding fragment thereof;
the second antibody is selected from the group consisting of the following antibodies or antigen-binding fragment thereof as defined in claim 2: (1a), (1b), (1c), (1e), (1f), and (1g), preferably (1a), (1b) or (1f);
V) the first antibody is the antibody of (1e) of claim 2 or antigen-binding fragment thereof;
the second antibody is selected from the group consisting of the following antibodies or antigen-binding fragment thereof as defined in claim 2: (1a), (1b), (1c), (1d), (1f), and (1g), preferably (1b), (1c) or (1f), more preferably (1b);
VI) the first antibody is the antibody of (1f) of claim 2 or antigen-binding fragment thereof;
the second antibody is selected from the group of the following antibodies or antigen-binding fragments thereof as described in claim 2: (1a), (1b), (1c), (1d), (1e), and (1g); or
VII) the first antibody is the antibody of (1g) of claim 2 or antigen-binding fragment thereof;
the second antibody is selected from the group consisting of the following antibodies or antigen-binding fragment thereof as defined in claim 2: (1a), (1b), (1c), (1d), (1e), and (1f).

12. The composition or PINP detection kit according to claim 11, wherein:
the first antibody is selected from the group consisting of the following antibodies or antigen-binding fragments thereof as defined in claim 2 (a1) to (a4); the second antibody is selected from the group consisting of the following antibodies or antigen-binding fragments thereof as defined in claim 2 (b1) to (b3), (d1) to (d3), (e1) to (e3) and (f1) to (f2);
the first antibody is selected from the group consisting of the following antibodies or antigen-binding fragments thereof as defined in claim 2 (b1) to (b3); the second antibody is selected from the group consisting of the following antibodies or antigen-binding fragments thereof as defined in claim 2 (a1) to (a4), (c1) to (c3), (d1) to (d3), (e1) to (e3) and (g1) to (g2);
the first antibody is selected from the group consisting of the following antibodies or antigen-binding fragments thereof as defined in claim 2 (c1) to (c3); the second antibody is selected from the group consisting of the following antibodies or antigen-binding fragments thereof as defined in claim 2 (b1) to (b3), (e1) to (e3) and (f1) to (f2);
the first antibody is selected from the group consisting of the following antibodies or antigen-binding fragments thereof as defined in claim 2 (d1) to (d3); the second antibody is selected from the group consisting of the following antibodies or antigen-binding fragments thereof as defined in claim 2 (a1) to (a4), (b1) to (b3) and (f1) to (f2); or
the first antibody is selected from the group consisting of the following antibodies or antigen-binding fragments thereof as defined in claim 2 (e1) to (e3); the second antibody is selected from the group consisting of the following antibodies or antigen-binding fragments thereof as defined in claim 2 (b1) to (b3), (c1) to (c3) and (f1) to (f2);
preferably, the PINP detection kit is an ELISA kit; more preferably, the first antibody is a capture antibody, the second antibody is a detection antibody; alternatively, the first antibody is a detection antibody, the second antibody is a capture antibody.

13. A method for detecting presence or concentration of tPINP, especially PINP, in a sample, comprising: detecting by using the antibody or antigen binding fragment thereof according to any one of claims 1-6 or the PINP detection kit according to any one of claims 10-12.
preferably, the method comprises:
contacting the sample with two or more of the antibodies or antigen-binding fragments thereof, and
qualitatively or quantitatively detecting binding of the two or more antibodies or antigen-binding fragments thereof to PINP and different forms of existence thereof (e.g., a monomer of PINP);
the binding indicating the presence or concentration of PINP and different forms of existence in the sample, wherein,
the two or more antibodies or antigen-binding fragments thereof target two or more epitopes of the PINP and different forms of existence thereof;
more preferably, at least one of the two or more antibodies or antigen-binding fragments thereof is a capture antibody and rest is or are a detection antibody or detection antibodies;
preferably, the capture antibody is any of the first antibodies defined in claim 12;
preferably, the detection antibody is any of the second antibodies defined in claim 12.

14. A detection quality control or calibrator, comprising a PINP having an amino acid sequence of SEQ ID NO: 43;
preferably, the detection is ELISA immunoassay, such as double antibody sandwich ELISA immunoassay.

15. Use of the antibody or antigen-binding fragment thereof according to any one of claims 1-6, the composition or PINP detection kit according to any one of claims 10-12, or the method according to claim 13 in detecting presence or concentration of tPINP, especially PINP, in a sample.

16. Use of the antibody or antigen-binding fragment thereof according to any one of claims 1-6 in manufacturing of a PINP detection kit for detection of presence or concentration of tPINP, especially PINP, in a sample.
